(19) **Europäisches Patentamt**
**European Patent Office**
**Office européen des brevets**

(11) **EP 4 617 697 A1**

(12) **EUROPEAN PATENT APPLICATION**
published in accordance with Art. 153(4) EPC

(43) Date of publication:
**17.09.2025  Bulletin 2025/38**

(21) Application number: **23888602.2**

(22) Date of filing: **01.11.2023**

(51) International Patent Classification (IPC):
*G01R 33/09* (2006.01)      *A61B 5/242* (2021.01)
*G01R 33/02* (2006.01)      *G01R 33/10* (2006.01)
*H10N 50/10* (2023.01)      *H10N 59/00* (2023.01)

(52) Cooperative Patent Classification (CPC):
**A61B 5/242; G01R 33/02; G01R 33/09;**
**G01R 33/10; H10N 50/10; H10N 59/00**

(86) International application number:
**PCT/JP2023/039519**

(87) International publication number:
**WO 2024/101254 (16.05.2024 Gazette 2024/20)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority:  **09.11.2022  JP 2022179805**

(71) Applicant: **Sony Semiconductor Solutions**
**Corporation**
**Atsugi-shi, Kanagawa 243-0014 (JP)**

(72) Inventors:
• **HIGO, Yutaka**
  **Atsugi-shi, Kanagawa 243-0014 (JP)**
• **SEGAMI, Masahiro**
  **Atsugi-shi, Kanagawa 243-0014 (JP)**
• **HOSOMI, Masanori**
  **Atsugi-shi, Kanagawa 243-0014 (JP)**
• **SAKAI, Lui**
  **Atsugi-shi, Kanagawa 243-0014 (JP)**

(74) Representative: **MFG Patentanwälte**
**Meyer-Wildhagen Meggle-Freund**
**Gerhard PartG mbB**
**Amalienstraße 62**
**80799 München (DE)**

(54) **MAGNETIC DETECTING DEVICE, AND DECODING SYSTEM**

(57)    There is provided a magnetic detection device including a pixel array unit configured by pixel units including a magnetoresistive element and a detection unit being arranged in an array or configured by a basic array including a plurality of the pixel units being arranged in an array. The magnetoresistive element includes a fixed layer having a fixed magnetization direction, a nonmagnetic layer disposed on the fixed layer, and a storage layer disposed on the nonmagnetic layer. The detection unit detects an external magnetic field based on a change in a resistance value of the magnetoresistive element.

FIG.38

## Description

Field

The present disclosure relates to a magnetic detection device and a decoding system.

Background

Magnetic detection devices using principles such as the Hall effect and the magnetoresistance effect are widely used because of their ease of use. However, when biomagnetism involved in a brain activity, a heart activity, or the like is to be measured, since the biomagnetism is weak, in a magnetic detection device that has been generally used, it is difficult to say that its sensitivity is sufficient for these measurements. Thus, in the measurement of the biomagnetism involved in the brain activity, the heart activity, or the like, in general, it is common to use a SQUID (Superconducting Quantum Interference Device) magnetic detection device that makes use of the magnetic quantum effect.

However, in the SQUID magnetic detection device, since it is necessary to cool the SQUID magnetic detection device to a very low temperature, equipment increases in size and it is difficult to provide a simple structure. Therefore, there has been a demand for a magnetic detection device capable of performing measurement with high accuracy while having a simple structure. When biomagnetism involved in a brain activity, a heart activity, or the like is measured, it is desirable to perform measurement at a plurality of points of the head surface or the chest surface instead of performing measurement at one point of the head surface or the chest surface. Thus, Patent Literature 1 below proposes, for example, a magnetocardiogram measurement device (a magnetic detection device) capable of measuring a magnetocardiogram (a magnetic field due to a weak current generated when a heart muscle is expanded and contracted to send blood into a body) with higher accuracy by disposing a plurality of magnetoresistive elements in a matrix.

Citation List

Patent Literature

Patent Literature 1: JP 2020-156870 A

Summary

Technical Problem

Incidentally, in the magnetocardiogram measurement device (the magnetic detection device) proposed in Patent Literature 1 described above, input magnetic fields in triaxial directions are measured by disposing, along an X axis, a Y axis, and a Z axis, the magnetoresistive elements capable of measuring an input magnetic field (an external magnetic field) in a uniaxial direction. However, the magnetocardiogram measurement device proposed in Patent Literature 1 has a problem that it is difficult to avoid a structure being complicated and the positions of magnetic fields measured by the magnetoresistive elements corresponding to the axes do not coincide with one another.

Therefore, the present disclosure proposes a magnetic detection device and a decoding system capable that can measure input magnetic fields in a plurality of axial directions at matched positions in a space while having a simpler structure.

Solution to Problem

According to the present disclosure, there is provided a magnetic detection device including a pixel array unit. The pixel array unit is configured by pixel units including a magnetoresistive element and a detection unit being arranged in an array, or is configured by a basic array including a plurality of the pixel units being arranged in an array. In the magnetic detection device, the magnetoresistive element includes: a fixed layer having a fixed magnetization direction; a nonmagnetic layer disposed on the fixed layer; and a storage layer disposed on the nonmagnetic layer, and the detection unit detects an external magnetic field based on a change in a resistance value of the magnetoresistive element.

Furthermore, according to the present disclosure, there is provided a decoding system including: a magnetic detection device worn on a user; an encoder; and an arithmetic unit. In the decoding system, the magnetic detection device includes a pixel array unit, the pixel array unit is configured by pixel units including a magnetoresistive element and a detection unit being arranged in an array, or is configured by a basic array including a plurality of the pixel units being arranged in an array, the magnetoresistive element includes: a fixed layer having a fixed magnetization direction; a nonmagnetic layer disposed on the fixed layer; and a storage layer disposed on the nonmagnetic layer, the detection unit detects an external magnetic field based on a change in a resistance value of the magnetoresistive element, the encoder acquires the external magnetic field from the magnetic detection device as input data and calculates a feature vector based on the input data, and the arithmetic unit outputs, based on the feature vector, a thought of the user or a stimulus given to the user.

Brief Description of Drawings

FIG. 1 is a schematic diagram illustrating a schematic configuration example of a magnetoresistive element according to a first embodiment of the present disclosure.

FIG. 2 is a schematic diagram illustrating a more detailed configuration example of the magnetoresistive element according to the first embodiment of the present disclosure.

FIG. 3 is a schematic diagram illustrating another schematic configuration example of the magnetoresistive element according to the first embodiment of the present disclosure.

FIG. 4 is a diagram illustrating a direction of an input magnetic field with respect to a storage layer according to the first embodiment of the present disclosure.

FIG. 5 is a diagram illustrating a relationship between a direction of an input magnetic field and an output signal (a stay time difference) in the case in which an in-plane magnetization film is used in the storage layer according to the first embodiment of the present disclosure.

FIG. 6 is a plan view illustrating a schematic configuration example of the magnetoresistive element according to the first embodiment of the present disclosure.

FIG. 7 is a plan view illustrating a schematic configuration example of another magnetoresistive element according to the first embodiment of the present disclosure.

FIG. 8 is a plan view illustrating a schematic configuration example of still another magnetoresistive element according to the first embodiment of the present disclosure.

FIG. 9 is a plan view illustrating a schematic configuration example of still another magnetoresistive element according to the first embodiment of the present disclosure.

FIG. 10 is a plan view illustrating a schematic configuration example of still another magnetoresistive element according to the first embodiment of the present disclosure.

FIG. 11 is a plane layout diagram illustrating a first example of an array of magnetoresistive elements according to the first embodiment of the present disclosure, the first example being an array example of the magnetoresistive elements that detect an input magnetic field in one axis (an X axis) in a plane.

FIG. 12 is a plane layout diagram illustrating a second example of the array of the magnetoresistive elements according to the first embodiment of the present disclosure, the second example being an array example of the magnetoresistive elements that detect an input magnetic field in one axis (a Y axis).

FIG. 13 is a plane layout diagram illustrating a third example of the array of the magnetoresistive elements according to the first embodiment of the present disclosure, the third example being an array example of the magnetoresistive elements that detect an input magnetic field in one axis (a Z axis).

FIG. 14 is a plane layout diagram illustrating a fourth example of the array of the magnetoresistive elements according to the first embodiment of the present disclosure, the fourth example being an array example of the magnetoresistive elements that detect input magnetic fields in two axes (the X axis and the Y axis) in a plane.

FIG. 15 is a plane layout diagram illustrating a fifth example of the array of the magnetoresistive elements according to the first embodiment of the present disclosure, the fifth example being an array example of the magnetoresistive elements that detect input magnetic fields in two axes (the X axis and the Y axis) in a plane.

FIG. 16 is a plane layout diagram illustrating a sixth example of the array of the magnetoresistive elements according to the first embodiment of the present disclosure, the sixth example being an array example of the magnetoresistive elements that detect input magnetic fields in three axes (the X axis, the Y axis, and the X axis).

FIG. 17 is a plane layout diagram illustrating a seventh example of the array of the magnetoresistive elements according to the first embodiment of the present disclosure, the seventh example being an array example of the magnetoresistive elements that detect input magnetic fields in three axes (X axis, Y axis, and X axis).

FIG. 18 is a plane layout diagram illustrating an eighth example of the array of the magnetoresistive elements according to the first embodiment of the present disclosure, the eighth example being an array example of the magnetoresistive elements that detect input magnetic fields in two axes (the X axis and the X axis).

FIG. 19 is a plane layout diagram illustrating a ninth example of the array of the magnetoresistive elements according to the first embodiment of the present disclosure, the ninth example being an array example of the magnetoresistive elements that detect input magnetic fields in two axes (the Y axis and the X axis).

FIG. 20 is a plane layout diagram illustrating a tenth example of the array of the magnetoresistive elements according to the first embodiment of the present disclosure, the tenth example being an array example of the magnetoresistive elements that detect input magnetic fields in three axes (the X axis, the Y axis, and the X axis).

FIG. 21 is a perspective view illustrating an eleventh example of the array of the magnetoresistive elements according to the first embodiment of the present disclosure, the eleventh example being an array example of the magnetoresistive elements that detect input magnetic fields in three axes (the X axis, the Y axis, and the X axis).

FIG. 22 is a process cross-sectional view illustrating an example of a method of manufacturing a magnetoresistive element according to the first embodiment of the present disclosure (part 1).

FIG. 23 is a process cross-sectional view illustrating the example of the method of manufacturing the magnetoresistive element according to the first embodiment of the present disclosure (part 2).

FIG. 24 is a process cross-sectional view illustrating the example of the method of manufacturing the magnetoresistive element according to the first embodiment of the present disclosure (part 3).

FIG. 25 is a process cross-sectional view illustrating the example of the method of manufacturing the magnetoresistive element according to the first embodiment of the present disclosure (part 4).

FIG. 26 is a process cross-sectional view illustrating the example of the method of manufacturing the magnetoresistive element according to the first embodiment of the present disclosure (part 5).

FIG. 27 is a process cross-sectional view illustrating the example of the method of manufacturing the magnetoresistive element according to the first embodiment of the present disclosure (part 6).

FIG. 28 is a process cross-sectional view illustrating the example of the method of manufacturing the magnetoresistive element according to the first embodiment of the present disclosure (part 7).

FIG. 29 is a process cross-sectional view illustrating the example of the method of manufacturing the magnetoresistive element according to the first embodiment of the present disclosure (part 8).

FIG. 30 is a process cross-sectional view illustrating the example of the method of manufacturing the magnetoresistive element according to the first embodiment of the present disclosure (part 9).

FIG. 31 is a process cross-sectional view illustrating another example of the method of manufacturing the magnetoresistive element according to the first embodiment of the present disclosure (part 1).

FIG. 32 is a process cross-sectional view illustrating the other example of the method of manufacturing the magnetoresistive element according to the first embodiment of the present disclosure (part 2).

FIG. 33 is a process cross-sectional view illustrating the other example of the method of manufacturing the magnetoresistive element according to the first embodiment of the present disclosure (part 3).

FIG. 34 is a process cross-sectional view illustrating the other example of the method of manufacturing the magnetoresistive element according to the first embodiment of the present disclosure (part 4).

FIG. 35 is a process cross-sectional view illustrating another example of the method of manufacturing the magnetoresistive element according to the first embodiment of the present disclosure (part 5).

FIG. 36 is a process cross-sectional view illustrating the other example of the method of manufacturing the magnetoresistive element according to the first embodiment of the present disclosure (part 6).

FIG. 37 is a process cross-sectional view illustrating the other example of the method of manufacturing the magnetoresistive element according to the first embodiment of the present disclosure (part 7).

FIG. 38 is a block diagram illustrating a schematic configuration example of a magnetic detection device according to the first embodiment of the present disclosure.

FIG. 39 is a block diagram illustrating a first example of an array of pixel units according to the first embodiment of the present disclosure.

FIG. 40 is a block diagram illustrating a second example of the array of pixel units according to the first embodiment of the present disclosure.

FIG. 41 is a block diagram illustrating a third example of the array of pixel units according to the first embodiment of the present disclosure.

FIG. 42 is a block diagram illustrating a fourth example of the array of pixel units according to the first embodiment of the present disclosure.

FIG. 43 is a flowchart illustrating a first example of a write operation of the magnetic detection device according to the first embodiment of the present disclosure.

FIG. 44 is a timing chart illustrating a first example of a write operation of a magnetic field measuring device according to the first embodiment of the present disclosure.

FIG. 45 is a flowchart illustrating a second example of the write operation of the magnetic field measuring device according to the first embodiment of the present disclosure.

FIG. 46 is a timing chart illustrating a second example of the write operation of the magnetic field measuring device according to the first embodiment of the present disclosure.

FIG. 47 is a diagram illustrating a first output data format example of the magnetic field measuring device according to the first embodiment of the present disclosure.

FIG. 48 is a diagram illustrating a second output data format example of the magnetic field measuring device according to the first embodiment of the present disclosure.

FIG. 49 is a diagram illustrating a third output data format example of the magnetic field measuring device according to the first embodiment of the present disclosure.

FIG. 50 is a diagram illustrating a first circuit configuration example of a pixel unit according to the first embodiment of the present disclosure.

FIG. 51 is a diagram illustrating a second circuit configuration example of the pixel unit according to the first embodiment of the present disclosure.

FIG. 52 is a diagram illustrating a third circuit configuration example of the pixel unit according to the first embodiment of the present disclosure.

FIG. 53 is a diagram illustrating a fourth circuit configuration example of the pixel unit according to the first embodiment of the present disclosure.

FIG. 54 is a diagram illustrating a fifth circuit configuration example of the pixel unit according to the first embodiment of the present disclosure.

FIG. 55 is a diagram illustrating a sixth circuit configuration example of the pixel unit according to the first embodiment of the present disclosure.

FIG. 56 is a diagram illustrating a first circuit configuration example of digitizing means according to the first embodiment of the present disclosure.

FIG. 57 is a diagram illustrating a second circuit configuration example of the digitizing means according to the first embodiment of the present disclosure.

FIG. 58 is a diagram illustrating a third circuit configuration example of the digitizing means according to the first embodiment of the present disclosure.

FIG. 59 is a diagram illustrating a fourth circuit configuration example of the digitizing means according to the first embodiment of the present disclosure.

FIG. 60 is a diagram illustrating a fifth circuit configuration example of the digitizing means according to the first embodiment of the present disclosure.

FIG. 61 is a diagram illustrating a sixth circuit configuration example of the digitizing means according to the first embodiment of the present disclosure.

FIG. 62 is a diagram illustrating an operation example of a sixth circuit configuration example of the digitizing means according to the first embodiment of the present disclosure.

FIG. 63 is a diagram illustrating a seventh circuit configuration example of the digitizing means according to the first embodiment of the present disclosure.

FIG. 64 is a diagram illustrating a first configuration example of a decoding system according to a second embodiment of the present disclosure.

FIG. 65 is a diagram illustrating a use example of a first configuration of the decoding system according to the second embodiment of the present disclosure.

FIG. 66 is a diagram illustrating a second configuration example of the decoding system according to the second embodiment of the present disclosure.

Description of Embodiments

Preferred embodiments of the present disclosure are explained in detail below with reference to the accompanying drawings. Note that, in the present specification and the drawings, components having substantially the same functional configurations are denoted by the same reference numerals and signs, whereby redundant explanation of the components is omitted.

Note that the explanation is made in the following order.

1. First embodiment
1.1 Configuration examples of magnetoresistive element
1.2 Variation examples of magnetoresistive element
1.3 Configuration examples of array of magnetoresistive elements
1.4 Manufacturing method example
1.5 Configuration example of magnetic detection device
1.6 Operation examples of magnetic detection device
1.7 Output data format examples
1.8 Circuit configuration examples of pixel unit
1.9 Digitizing means examples
2. Second embodiment
2.1 First configuration example of decoding system

2.2 Second configuration example of decoding system

3. Supplement

<<1. First embodiment>>

<1.1 Configuration examples of magnetoresistive element>

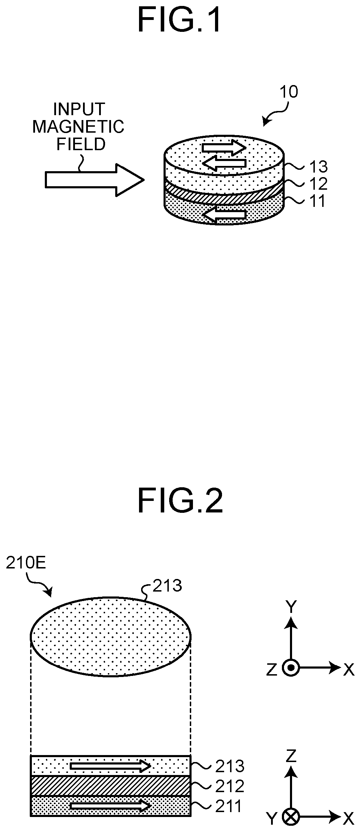

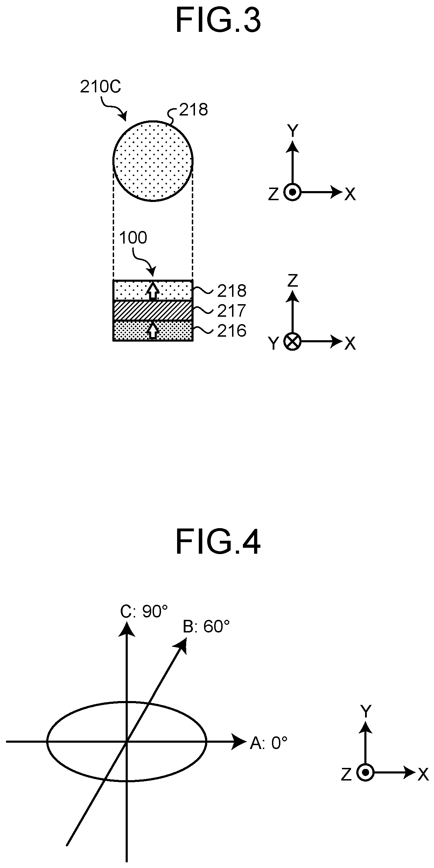

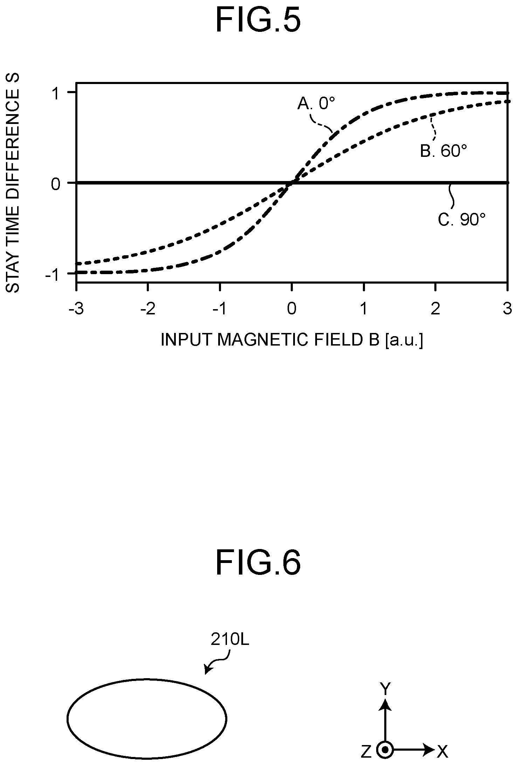

First, a magnetoresistive element 10 and a magnetic detection device 20 according to a first embodiment of the present disclosure are explained in detail with reference to FIG. 1 to FIG. 5. FIG. 1 is a schematic diagram illustrating a schematic configuration example of the magnetoresistive element 10 according to the present embodiment. FIG. 2 is a schematic diagram illustrating a more detailed configuration example of the magnetoresistive element 10 according to the present embodiment. FIG. 3 is a schematic diagram illustrating another schematic configuration example of the magnetoresistive element 10 according to the present embodiment. Further, FIG. 4 is a diagram illustrating a direction of an input magnetic field with respect to a storage layer 13 according to the present embodiment. FIG. 5 is a diagram illustrating a relationship between a direction of an input magnetic field (an external magnetic field) and an output signal (a stay time difference) in a case in which an in-plane magnetization film is used in the storage layer 13 according to the present embodiment.

First, the magnetoresistive element 10 according to the present embodiment is explained with reference to FIG. 1. As illustrated in FIG. 1, the magnetoresistive element 10 includes, for example, a magnetization fixed layer (a fixed layer) 11 whose magnetization direction is fixed, a storage layer 13 whose magnetization direction changes according to an input magnetic field, and a nonmagnetic layer 12 disposed between the magnetization fixed layer 11 and the storage layer 13. Layers of the magnetoresistive element 10 are explained below.

The magnetization fixed layer 11 can be a layer in which the magnetization direction is fixed by combining a ferromagnetic material such as a cobalt iron (CoFe) alloy with an antiferromagnetic material such as a platinum manganese (PtMn) alloy or an iridium manganese (IrMn) alloy. Alternatively, the magnetization fixed layer 11 can be formed in a structure in which at least two ferromagnetic layers are laminated with an extremely thin ruthenium (Ru) layer, iridium (Ir) layer, or the like. With such a configuration, the magnetization direction of the magnetization fixed layer 11 is fixed. Furthermore, with such a configuration, since the ferromagnetic layers are coupled antiparallel to each other, a leakage magnetic field from the magnetization fixed layer 11 can be reduced.

The storage layer 13 is formed of a magnetic material having weak magnetic anisotropy such as a CoFe alloy, a nickel-iron (NiFe) alloy, or a cobalt-iron boron (CoFeB) alloy so as to easily change with respect to the input magnetic field.

The nonmagnetic layer 12 can be formed of an insulator such as alumina ($Al_2O_3$) or magnesium oxide (MgO). By using such an insulator, feeding an electric current in a film surface perpendicular direction, and utilizing the tunnel magneto resistance (TMR) effect, a large resistance change can be caused in the magnetoresistive element 10.

Furthermore, as illustrated in FIG. 1, in the magnetoresistive element 10 according to the present embodiment, a magnetic anisotropic easy axis (hereinafter simply referred to as easy axis) of the storage layer 13 is configured to be parallel to the magnetization direction of the magnetization fixed layer 11. Then, when the easy axis of the storage layer 13 and the magnetization direction of the magnetization fixed layer 11 are made parallel, the magnetization direction of the storage layer 13 is limited to either a state close to parallel to the magnetization direction of the magnetization fixed layer 11 (hereinafter simply referred to as parallel state) or a state close to antiparallel to the magnetization direction (hereinafter simply referred to as antiparallel state) depending on the direction and the magnitude of the input magnetic field. Furthermore, since the magnetization direction of the storage layer 13 is limited to the two states, the resistance of the magnetoresistive element 10 has two states (high resistance and low resistance).

FIG. 2 and FIG. 3 are schematic diagrams illustrating more detailed configuration examples of the magnetoresistive element 10 according to the present embodiment. The easy axis of the storage layer 13 illustrated in FIG. 1 is in an in- film plane direction in some cases and is in a film surface perpendicular direction in other cases. When the magnetization direction of the storage layer 13 is equal to the easy axis, the magnetization is stabilized. Specifically, FIG. 2 is a diagram illustrating an example of a magnetoresistive element 210E in which an in-plane magnetization film that stabilizes when the magnetization direction is in the film plane is used in a storage layer 213. An upper part of FIG. 2 illustrates a top view of the magnetoresistive element 210E and a lower part of FIG. 2 illustrates a vertical cross-sectional view parallel to the long axis direction (an X direction in this example) of the magnetoresistive element 210E. On the other hand, FIG. 3 is a diagram illustrating a magnetoresistive element 210C in which a perpendicular magnetization film that stabilizes when the magnetization direction is in a film surface perpendicular direction (a Z direction in this example) is used in a storage layer 218. An upper part of FIG. 3 illustrates a top view of the magnetoresistive element 210C and a lower part of FIG. 3 illustrates a vertical cross-sectional view of the magnetoresistive element 210C. In the examples illustrated in FIG. 2 and FIG. 3, as in FIG. 1, whereas the magnetization directions of the storage layers 213 and 218 are variable according to the input magnetic field, the magnetization directions of magnetization fixed layers 211 and 216 are fixed.

As illustrated in FIG. 2, the upper surface shape of the magnetoresistive element 210E in which the in-plane magnetization film is used in the storage layer 213 has a line symmetrical shape having a longitudinal direction in the in-plane direction and having, as an axis, a straight line perpendicular to the longitudinal direction and passing a center point. FIG. 2 illustrates, for example, a case in which the upper surface shape of the magnetoresistive element 210E is an elliptical shape having a major axis in the in-plane direction. In this case, the magnetization direction of the storage layer 213 is easily directed in the long axis direction. That is, it can be said that the longitudinal direction is an easy axis direction in which the magnetization direction of the storage layer 213 is easily directed. Conversely, in the example illustrated in FIG. 2, the magnetization direction of the storage layer 213 is less easily directed in the short direction, and this direction is referred to as a difficult axis direction.

However, the present embodiment is not limited to this and the upper surface shape of the magnetoresistive element 210E can be changed as appropriate to, for example, a polygon having a longitudinal direction in the in-plane direction such as a rectangle. The magnetization direction of the magnetization fixed layer 211 of the magnetoresistive element 210E is set in a direction parallel to the longitudinal direction. Furthermore, in the example illustrated in FIG. 2, a nonmagnetic layer 212 is disposed between the magnetization fixed layer 211 and the storage layer 213.

On the other hand, as illustrated in FIG. 3, the upper surface shape of the magnetoresistive element 210C in which the perpendicular magnetization film is used in the storage layer 218 has a point symmetrical shape not having a longitudinal direction in the in-plane direction and having a center point as an axis. FIG. 3 illustrates, for example, a case in which the upper surface shape of the magnetoresistive element 210E is a circular shape not having a major axis in the in-plane direction. However, the present embodiment is not limited to this and the upper surface shape of the magnetoresistive element 210E can be changed as appropriate to, for example, a polygon not having a longitudinal direction in the in-plane direction such as a square or a regular hexagon. The magnetization direction of the magnetization fixed layer 216 of the magnetoresistive element 210C is set in a direction perpendicular to formation surfaces of the layers. Furthermore, in the example illustrated in FIG. 3, a nonmagnetic layer 217 is disposed between the magnetization fixed layer 216 and the storage layer 218.

Referring back to FIG. 1, the magnetization direction of the storage layer 13 is explained more in detail. When the volume of the storage layer 13 is large, the magnetization of the storage layer 13 stabilizes in either the parallel state or the antiparallel state. However, as the volume of the storage layer 13 is reduced, the magnetization of the storage layer 13 transitions between the parallel state and the antiparallel state because of the effect of thermal fluctuation.

Here, a thermal stability index $\Delta_0$ of a state of the storage layer 13 is represented by the following Expression (1) using magnetic anisotropy energy $K_u$, volume V of a magnetic body, temperature T, and a Boltzmann constant $k_B$.

$$\Delta_0 = K_u V / k_B T \qquad \cdots (1)$$

Therefore, a reversal probability P of the magnetization direction of the storage layer 13 reversing during a time t can be represented by the following Expression (2). In Expression (2), $\tau_0$ is a relaxation constant.

$$P = 1 - exp\{-t/\tau_0 \cdot exp(-\Delta_0)\} \qquad \cdots (2)$$

When an input magnetic field is applied to the magnetoresistive element 10, a thermal stability index $\Delta_+$ in a state ($S_+$) close to the direction of the input magnetic field and a thermal stability index $\Delta_-$ in a state ($S_-$) far from the direction of the input magnetic field are different from each other. Specifically, the thermal stability index $\Delta_+$ in the state close to the direction of the input magnetic field ($S_+$) is larger than the thermal stability index $\Delta_-$ in the state far from the direction of the input magnetic field ($S_-$) ($\Delta_+ > \Delta_-$). That is, when the input magnetic field is applied to the magnetoresistive element 10, a difference occurs between the thermal stability index $\Delta_+$ and the thermal stability index $\Delta_-$ and a difference occurs between the reversal probability P from the state $S_+$ to the state $S_-$ and the reversal probability P from the state $S_-$ to the state $S_+$. Therefore, a difference occurs between a stay time in which the magnetization of the storage layer 13 is in the state $S_+$ and a stay time in which the magnetization of the storage layer 13 is in the state $S_-$.

As a result of various studies, the present inventors uniquely found that a value S obtained by dividing the difference between the stay time in the state $S_+$ and the stay time in the state $S_-$ by a sum of the stay time in the state $S_+$ and the stay time in the state $S_-$ (hereinafter, in the present specification, the value S is referred to as "stay time difference") can be represented by the following Expression (3).

$$S = tanh(M_s V B_\parallel / k_B T) \qquad \cdots (3)$$

In the above Expression (3), $M_s$ represents saturation magnetization of the storage layer 13 and $B_\parallel$ represents an easy axis component of an input magnetic field B.

By using the above Expression (3), it is possible to detect only a component along an easy axis direction from an input magnetic field having any angle by obtaining the stay time difference S.

FIG. 4 and FIG. 5 illustrate a relationship between the direction of the input magnetic field and an output signal (a stay time difference) S in the case in which the in-plane magnetization film is used in the storage layer 213. In FIG. 4 and FIG. 5, A indicates a case in which the direction of an input magnetic field B is parallel to the easy axis, B indicates a case in which the direction of the input magnetic field B is inclined by 60° with respect to the easy axis, and C indicates a case in which the direction of the input magnetic field B is inclined by 90° with respect to the easy axis, that is, perpendicular. As illustrated in FIG. 5, in A in which the direction of the input magnetic field B is equal to the easy axis, a change in the output signal S is the largest, in other words, sensitivity is the highest. Conversely, in C in which the direction of the input magnetic field B is perpendicular to the easy axis, that is, equal to a difficult axis, the change in the output signal S is zero, in other words, the sensitivity is zero.

The magnetoresistive elements 210E and 210C configured to have such an easy axis have directivity in the sensitivity to the input magnetic field. Therefore, in the present embodiment, by combining magnetoresistive elements having different directions of easy axes, not only the magnitude of the input magnetic field but also the direction of the input magnetic field can be detected.

<1.2 Variation examples of magnetoresistive element>

Subsequently, variation examples of the magnetoresistive element according to the present embodiment are explained with reference to FIG. 6 to FIG. 10. FIG. 6 to FIG. 10 are top views illustrating a part of the variation examples of the magnetoresistive element according to the present embodiment.

FIG. 6 is a top view illustrating a planar configuration example of a magnetoresistive element (a first magnetoresistive element) 210L in which an easy axis is parallel to the lateral direction (the X direction) (a first direction) in the magnetoresistive element 210E in which the in-plane magnetization film having the easy axis in the in-plane direction is used in the storage layer 213. According to the magnetoresistive element 210L in which the easy axis is parallel to the lateral direction (the X direction), the component in the X direction in the input magnetic field B can be detected with high sensitivity.

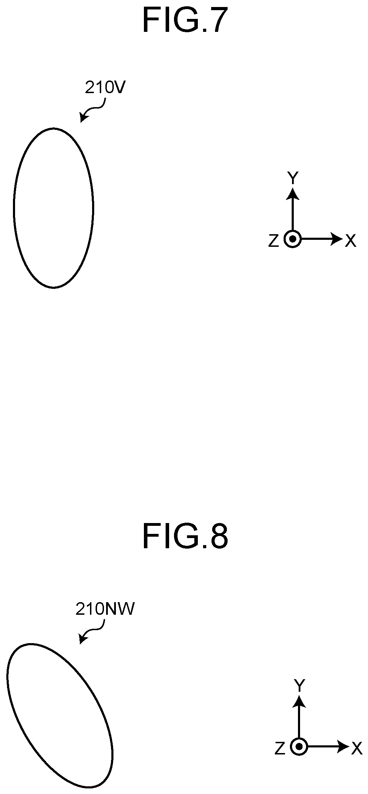

FIG. 7 is a top view also illustrating a planar configuration example of a magnetoresistive element (a second magnetoresistive element) 210V in which an easy axis is parallel to the longitudinal direction (the Y direction) (a second direction) in the magnetoresistive element 210E. With the magnetoresistive element 210V in which the easy axis is parallel to the longitudinal direction (the Y direction), a component in the Y direction in the input magnetic field B can be detected with high sensitivity.

FIG. 8 is a top view also illustrating a planar configuration example of a magnetoresistive element 210NW parallel to a direction (hereinafter also referred to as -XY direction or left oblique direction) in which an easy axis is inclined counterclockwise by 135° with respect to the X direction in the magnetoresistive element 210E. The magnetoresistive element NW is a variation for complementing magnetic field detection in the in-plane direction by the magnetoresistive element 210L and the magnetoresistive element 210V and can detect a component in the left oblique direction in the input magnetic field B with high sensitivity.

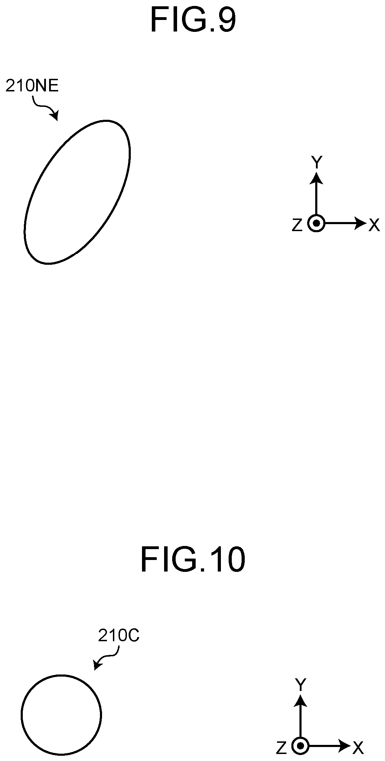

FIG. 9 is a top view also illustrating a planar configuration example of a magnetoresistive element 210NE parallel to a direction in which an easy axis is inclined counterclockwise by 45° with respect to the X direction (hereinafter also referred to as +XY direction or right oblique direction) in the magnetoresistive element 210E. Like the magnetoresistive element NW, the magnetoresistive element NE is a variation for complementing magnetic field detection in the in-plane direction by the magnetoresistive element 210L and the magnetoresistive element 210V and can detect a component in the right oblique direction in the input magnetic field B with high sensitivity.

FIG. 10 is a top view illustrating a planar configuration example of a magnetoresistive element (a third magnetoresistive element) 210C in which a perpendicular magnetization film having an easy axis in the perpendicular direction (the Z direction) (a third direction) is used in the storage layer 218. With the magnetoresistive element 210C in which an easy axis is parallel to the vertical direction (the Z direction), a component in the vertical direction (the Z direction) in the input magnetic field B can be detected with high sensitivity.

In the present embodiment, it is possible to detect the direction of the input magnetic field with high sensitivity by combining, as appropriate, the magnetoresistive elements 210L, 210V, 210NW, 210NE, and 210C having different easy axis directions explained above.

Note that the present embodiment is not limited to the magnetoresistive elements having the forms illustrated in FIG. 6 to FIG. 10. For example, a magnetoresistive element in which an easy axis is parallel to a direction inclined at an angle other than the angles illustrated in FIG. 6 to FIG. 10 may be used.

<1.3 Configuration examples of array of magnetoresistive elements>

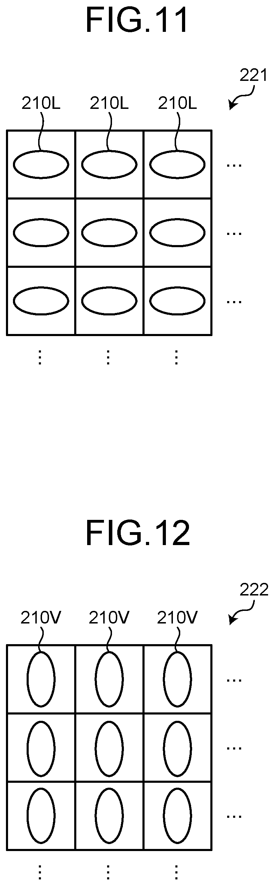

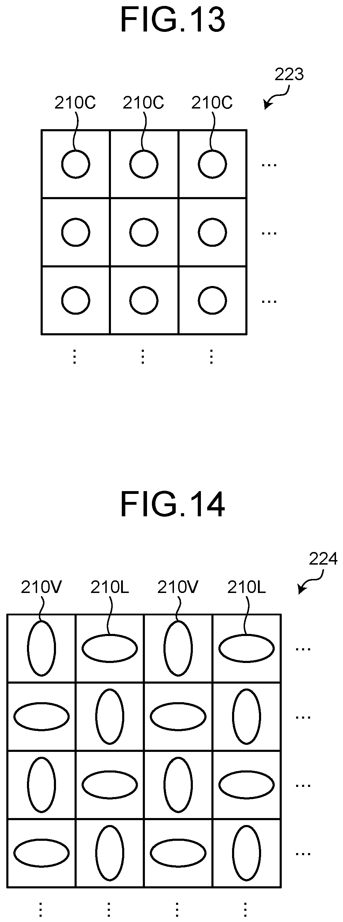

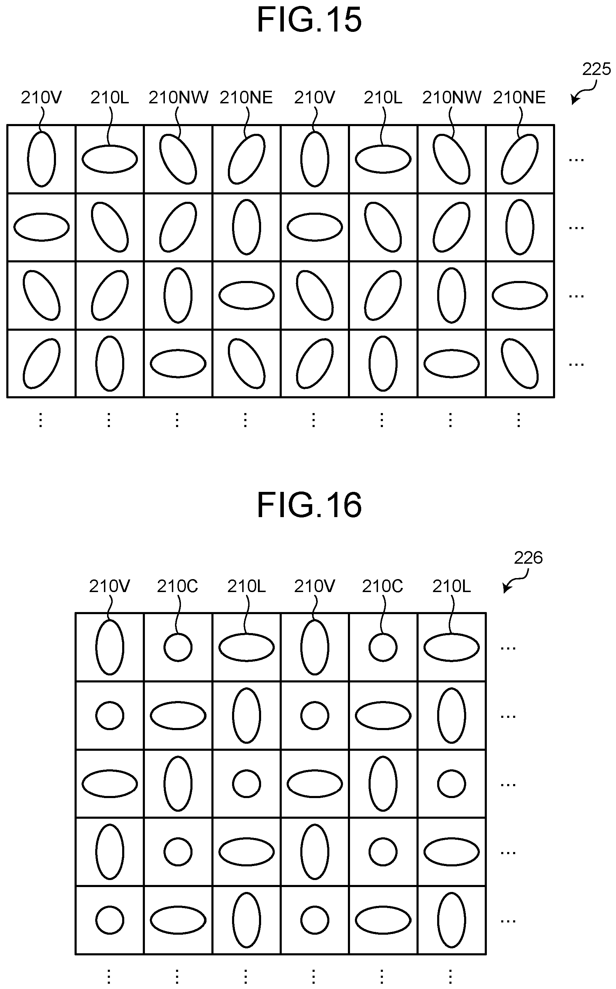

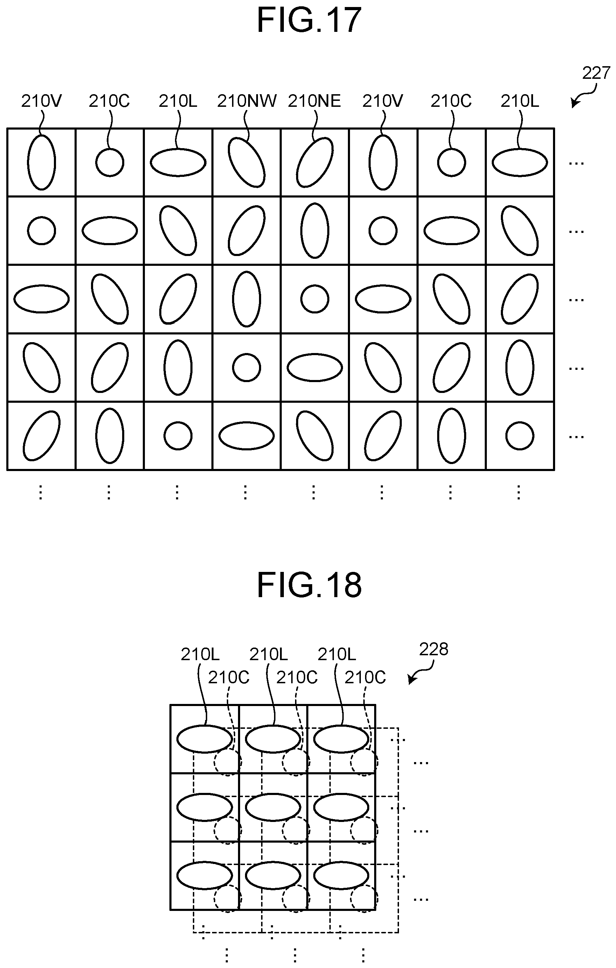

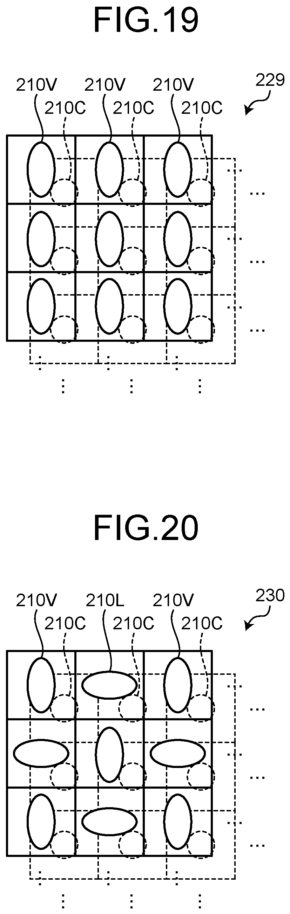

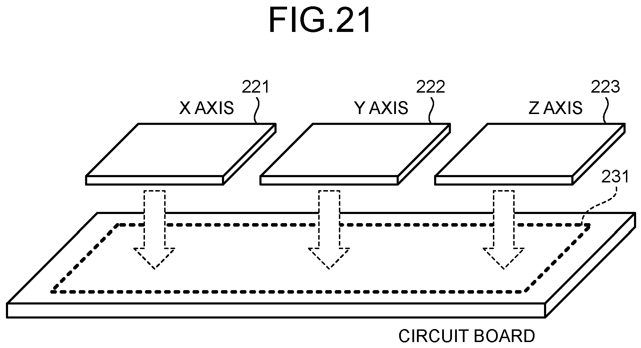

Subsequently, examples of an array of magnetoresistive elements according to the present embodiment are explained with reference to FIG. 11 to FIG. 21. FIG. 11 is a plane layout diagram illustrating a first example of the array of the magnetoresistive elements according to the present embodiment, the first example being an array example of magnetoresistive elements that detect an input magnetic field in one axis (an X axis) in a plane. FIG. 12 is a plane layout diagram illustrating a second example of the array of the magnetoresistive elements according to the present embodiment, the second example being an array example of magnetoresistive elements that detect an input magnetic field in one axis (a Y axis). FIG. 13 is a plane layout diagram illustrating a third example of the array of the magnetoresistive elements according to the present embodiment, the third example being an array example of magnetoresistive elements that detect an input magnetic field in one axis (a Z axis). FIG. 14 is a plane layout diagram illustrating a fourth example of the array of the magnetoresistive elements according to the present embodiment, the fourth example being an array example of magnetoresistive elements that detect input magnetic fields in two axes (the X axis and the Y axis) in a plane. FIG. 15 is a plane layout diagram illustrating a fifth example of the array of the magnetoresistive elements according to the present embodiment, the fifth example being an array example of magnetoresistive elements that detect input magnetic fields in two axes (the X axis and the Y axis) in a plane. FIG. 16 is a plane layout diagram illustrating a sixth example of the array of the magnetoresistive elements according to the present embodiment, the sixth example being an array example of magnetoresistive elements that detect input magnetic fields in three axes (the X axis, the Y axis, and the X axis). FIG. 17 is a plane layout diagram illustrating a seventh example of the array of the magnetoresistive elements according to the present embodiment, the seventh example being an array example of magnetoresistive elements that detect input magnetic fields in three axes (the X axis, the Y axis, and the X axis). FIG. 18 is a plane layout diagram illustrating an eighth example of the array of the magnetoresistive elements according to the present embodiment, the eighth example being an array example of magnetoresistive elements that detect input magnetic fields in two axes (the X axis and the X axis). FIG. 19 is a plane layout diagram illustrating a ninth example of the array of the magnetoresistive elements according to the present embodiment, the ninth example being an array example of magnetoresistive elements that detect input magnetic fields in two axes (the Y axis and the X axis). FIG. 20 is a plane layout diagram illustrating a tenth example of the array of the magnetoresistive elements according to the present embodiment, the tenth example being an array example of magnetoresistive elements that detect input magnetic fields in three axes (the X axis, the Y axis, and the X axis). FIG. 21 is a perspective view illustrating an eleventh example of the array of the magnetoresistive elements according to the present embodiment, the eleventh example being an array example of magnetoresistive elements that detect input magnetic fields in three axes (the X axis, the Y axis, and the X axis).

As explained above, in the present embodiment, the direction and the magnitude of the input magnetic field B are detected with high sensitivity by measuring the stay time difference (the output signal) S. However, for a significant stay time difference to occur, it is necessary that transition from the state $S_+$ to the state $S_-$ and transition from the state $S_-$ to the state $S_+$ occur at a certain frequency or more. Since these transitions probabilistically occur, there is a large temporal fluctuation in the stay time difference S. Therefore, in the present embodiment, it is effective to increase the number of transitions per observation time, that is, to reduce $\Delta_0$ in order to allow transitions to reliably occur and reduce fluctuation. In the present embodiment, for example, an average inversion time is preferably set to 10 milliseconds or less.

Furthermore, in the present embodiment, in order to reduce the influence of the fluctuation, the number of magnetoresistive elements only has to be increased to average states. For example, by disposing the magnetoresistive elements in series or in parallel and measuring information concerning a difference between the number of the magnetoresistive elements in a parallel state and the number of the magnetoresistive elements in an antiparallel state as a resistance value of an aggregate of the magneto resistive elements, it is possible to reduce the influence of the fluctuation. Furthermore, for example, the influence of the fluctuation can also be reduced by integrating the resistance value as an electric signal in a predetermined time length or allowing the fluctuation to pass a low pass filter circuit that removes a high frequency component.

In the present embodiment, when the aggregate of the magnetoresistive elements is configured, the configuration may be a configuration in which a plurality of magnetoresistive elements are connected in series, a configuration in which the magnetoresistive elements are connected in parallel, or a configuration in which the series connection and the parallel connection are combined.

Note that, in FIG. 11 to FIG. 20 used in the following explanation, only magnetoresistive elements configuring an array of magnetoresistive elements are described. However, it is assumed that the magnetoresistive elements are connected to one another by not-illustrated wires and circuits necessary for detecting a magnetic field. Note that details of these wires and circuits are explained below.

FIG. 11 is a plane layout diagram illustrating an array 221 of magnetoresistive elements of the first example according to the present embodiment, the array 221 being an array example of magnetoresistive elements that detect an input magnetic field in the X axis. As illustrated in FIG. 11, in the first example, magnetoresistive elements 210L having high sensitivity to a magnetic field component in the X direction are arrayed. As explained above, by disposing the magnetoresistive elements 210L that detect the magnetic field component in the X direction with high sensitivity, it is possible to detect the magnitude of the input magnetic field B in the X with high sensitivity.

FIG. 12 is a plane layout diagram illustrating an array 222 of magnetoresistive elements of the second example according to the present embodiment, the array 222 being an array example of magnetoresistive elements that detect an input magnetic field in the Y axis. As illustrated in FIG. 12, in the second example, magnetoresistive elements 210V having high sensitivity to a magnetic field component in the Y direction are arrayed. As explained above, by disposing the magnetoresistive elements 210V that detect the magnetic field component in the Y direction with high sensitivity, it is possible to detect the magnitude of the input magnetic field B in the Y direction with high sensitivity.

FIG. 13 is a plane layout diagram illustrating an array 223 of magnetoresistive elements of the third example according to the present embodiment, the array 223 being an array example of magnetoresistive elements that detect an input magnetic field in the Z axis. As illustrated in FIG. 13, in the third example, magnetoresistive elements 210C having high sensitivity to a magnetic field component in the Z direction are arrayed. As explained above, by disposing the magnetoresistive elements 210C that detect the magnetic field component in the Z direction with high sensitivity, it is possible to detect the magnitude of the input magnetic field B in the Z direction with high sensitivity.

FIG. 14 is a plane layout diagram illustrating an array 224 of magnetoresistive elements of the fourth example according to the present embodiment, the array 224 being an array example of magnetoresistive elements that detect input magnetic fields in two axes (the X axis and the Y axis) in a plane. As illustrated in FIG. 14, in the fourth example, the magnetoresistive elements 210L having high sensitivity to a magnetic field component in the X direction and the magnetoresistive elements 210V having high sensitivity to a magnetic field component in the Y direction are alternately arrayed in a checkered pattern. As explained above, by disposing the magnetoresistive elements 210L and the magnetoresistive elements 210V without deviation, it is possible to detect the magnitude and the direction of the input magnetic field B in the in-plane direction with high sensitivity. Note that, in the example, an array pattern is not limited to the pattern illustrated in FIG. 14. In the example, as long as the magnetoresistive elements 210L and 210V are equally arrayed as a whole, the array pattern can be changed as appropriate to, for example, array the magnetoresistive elements 210L and 210V in every other row or every other column.

FIG. 15 is a plane layout diagram illustrating an array 225 of magnetoresistive elements of the fifth example according to the present embodiment, the array 225 being an array example of magnetoresistive elements that detect input magnetic fields in two axes (the X axis and the Y axis) in a plane. As illustrated in FIG. 15, in fifth example, in addition to the magnetoresistive elements 210L and 210V, magnetoresistive elements 210NW having high sensitivity to a magnetic field component in the -XY direction and magnetoresistive elements 210NE having high sensitivity to a magnetic field component in the +XY direction are alternately arrayed. As explained above, by disposing the magnetoresistive elements 210L, 210V, 210NW, and 210NE without deviation, it is possible to detect the magnitude and the direction of the input magnetic field B in the in-plane direction with higher sensitivity. Note that, in the example, an array pattern is not limited to the pattern illustrated in FIG. 15. In the example, as long as the magnetoresistive elements 210L, 210V, 210NW, and 210NE are equally arrayed as a whole, the array pattern can be changed as appropriate to, for example, array the magnetoresistive elements 210L, 210V, 210NW, and 210NE in every other row or every other column.

FIG. 16 is a plane layout diagram illustrating an array 226 of magnetoresistive elements of the sixth example according to the present embodiment, the array 226 being an array example of magnetoresistive elements that detect input magnetic fields in three axes (the X axis, the Y axis, and the Z axis). As illustrated in FIG. 16, in the sixth embodiment, based on the array 224 of the magnetoresistive elements according to the fourth example, in addition to the magnetoresistive elements 210L and 210V according to the fourth example, the magnetoresistive elements 210C having high sensitivity to a magnetic field component in the Z direction are alternately arrayed. As explained above, by disposing, without deviation, the magnetoresistive elements 210L and 210V that detect the magnetic field component in the in-plane direction with high sensitivity and the magnetoresistive elements 210C that detect the magnetic field component in the Z direction with high sensitivity, it is possible to detect the magnitude and the direction of the input magnetic field B not only in the in-plane direction but also in the Z direction with high sensitivity. Note that, in the example, an array pattern is not limited to the pattern illustrated in FIG. 16. In the example, as long as the magnetoresistive elements 210L, 210V, and 210C are equally arrayed as a whole, the array pattern can be changed as appropriate to, for example, array the magnetoresistive elements 210L, 210V, and 210C in every other row or every other column.

FIG. 17 is a plane layout diagram illustrating an array 227 of magnetoresistive elements of the seventh example according to the present embodiment, the array 227 being an array example of magnetoresistive elements that detect input magnetic fields in three axes (the X axis, the Y axis, and the Z axis). As illustrated in FIG. 17, in the seventh example, based on the array 225 of the magnetoresistive elements according to the fifth example, in addition to the magnetoresistive elements 210L, 210V, 210NW, and 210NE according to the fifth example, the magnetoresistive elements 210C having high sensitivity to the magnetic field component in the Z direction are alternately arrayed. As explained above, by disposing, without deviation, the magnetoresistive elements 210L, 210V, 210NW, and 210NE that detect the magnetic field component in the in-plane direction with high sensitivity and the magnetoresistive element 210C that detects the magnetic field component in the Z direction with high sensitivity, it is possible to detect the magnitude and the direction of the input magnetic field B not only in the in-plane direction but also in the Z direction with higher sensitivity. Note that, in the example, an array pattern is not limited to the pattern illustrated in FIG. 17. In the example, as long as the magnetoresistive elements 210L, 210V, 210NW, 210NE, and 210C are equally arrayed as a whole, the array pattern can be changed as appropriate to, for example, array the magnetoresistive elements 210L, 210V, 210NW, 210NE, and 210C in every other row or every other column.

In the arrays of the magnetoresistive elements illustrated in FIG. 11 to FIG. 17, the magnetoresistive elements are formed on the same layer of a semiconductor wafer. On the other hand, in FIG. 18 to FIG. 20, magnetoresistive elements of different types are formed on different layers of a semiconductor wafer.

FIG. 18 is a plane layout diagram illustrating an array 228 of magnetoresistive elements of the eighth example according to the present embodiment, the array 228 being an array example of magnetoresistive elements that detect input magnetic fields in two axes (the X axis and the Z axis) in a plane. As illustrated in FIG. 18, in the eighth example, the magnetoresistive elements 210C having high sensitivity to the magnetic field component in the Z direction are arrayed in a first layer (in FIG. 18, indicated by a broken line). In a second layer (in FIG. 18, indicated by a solid line), the magnetoresistive elements 210L having high sensitivity to a magnetic field component in the X direction are arrayed. As explained above, by disposing the magnetoresistive elements 210L and 210C without deviation, it is possible to detect the magnitude and the direction of the input magnetic field B in an XZ in-plane direction with high sensitivity. Note that, in the example, the order of lamination of the first layer and the second layer may be opposite to the order illustrated in the figure.

FIG. 19 is a plane layout diagram illustrating an array 229 of magnetoresistive elements of the ninth example according to the present embodiment, the array 229 being an array example of magnetoresistive elements that detect input magnetic fields in two axes (the Y axis and the Z axis) in a plane. As illustrated in FIG. 19, in the ninth example, the magnetoresistive elements 210C having high sensitivity to the magnetic field component in the Z direction are arrayed in the first layer (in FIG. 19, indicated by a broken line). In the second layer (in FIG. 19, indicated by a solid line), the magnetoresistive elements 210V having high sensitivity to the magnetic field component in the Y direction are arrayed. As explained above, by disposing the magnetoresistive elements 210V and the magnetoresistive elements 210C without deviation, it is possible to detect the magnitude and the direction of the input magnetic field B in a YZ in-plane direction with high sensitivity. Note that, in the example, the order of lamination of the first layer and the second layer may be opposite to the order illustrated in the figure.

FIG. 20 is a plane layout diagram illustrating an array 230 of magnetoresistive elements of the tenth example according to the present embodiment, the array 230 being an array example that detect input magnetic fields in three axes (the X axis, the Y axis, and the Z axis). As illustrated in FIG. 20, in the tenth example, the magnetoresistive elements 210C having high sensitivity to the magnetic field component in the Z direction are arrayed in the first layer (in FIG. 20, indicated by a broken line). In the second layer (in FIG. 20, indicated by a solid line), the magnetoresistive elements 210L having high sensitivity to the magnetic field component in the X direction and the magnetoresistive elements 210V having high sensitivity to the magnetic field component in the Y direction are alternately arrayed in a checkered pattern. As explained above, by disposing, without deviation, the magnetoresistive elements 210L and 210V that detect the magnetic field component in the in-plane direction with high sensitivity and the magnetoresistive elements 210C that detect the magnetic field component in the Z direction with high sensitivity, it is possible to detect the magnitude and the direction of the input magnetic field B not only in the in-plane direction but also in the Z direction with high sensitivity. Note that, in the example, an array pattern of the second layer is not limited to the pattern illustrated in FIG. 20. In the example, as long as the magnetoresistive elements 210L and 210V are equally arrayed as a whole, the array pattern can be changed as appropriate to, for example, array the magnetoresistive elements 210L and 210V in every other row or every other column. In the example, the order of lamination of the first layer and the second layer may be the order opposite to the order illustrated in the figure.

In the present embodiment, by bonding the array of the one or more magnetoresistive elements illustrated in FIG. 11 to FIG. 20 to another circuit board, an array of magnetoresistive elements can also be configured anew.

For example, FIG. 21 is a perspective view illustrating an array 231 of magnetoresistive elements of the eleventh example according to the present embodiment, the array 231 being an array example of magnetoresistive elements that detect input magnetic fields in three axes (the X axis, the Y axis, and the Z axis). As illustrated in FIG. 21, in the eleventh example, the array 221 of the magnetoresistive elements that detect an input magnetic field in the X axis, the array 222 of the magnetoresistive elements that detect an input magnetic field in the Y axis, and the array 223 of the magnetoresistive elements that detect an input magnetic field in the Z axis are bonded to another circuit board to configure a new array 231 of magnetoresistive elements. In this way, it is possible to detect the magnitude and the direction of the input magnetic field B with higher sensitivity in the three axes (the X axis, the Y axis, and the Z axis). Note that the arrays of the magnetoresistive elements that detect the input magnetic fields in the X axis, the Y axis, and the Z axis may be obtained from the same wafer or may be obtained from different wafers. Furthermore, the array of the magnetoresistive elements that detect the input magnetic field in the Y axis may be obtained by rotating, by 90 degrees, the array of the magnetoresistive elements that detect the input magnetic field in the X axis.

Note that the present embodiment is not limited to the arrays of the magnetoresistive elements having the forms illustrated in FIG. 11 to FIG. 21.

<1.4 Manufacturing method example>

Subsequently, an example of a method of manufacturing a magnetoresistive element according to the present embodiment is explained.

Among the variations of the magnetoresistive elements explained above with reference to FIG. 6 to FIG. 10, all of the magnetoresistive elements 210L, 210V, 210NW, and 210NE (the variations of the magnetoresistive elements 210E) can be formed and processed in the same process because the in-plane magnetization film is used in the storage layer 213. On the other hand, since the perpendicular magnetization film is used in the storage layer 218, the magnetoresistive elements 210C cannot be formed in the same process as the process for the magnetoresistive elements 210E and needs to be formed and processed in another process.

Then, when the magnetoresistive elements 210E in which the in-plane magnetization film is used in the storage layer 213 and the magnetoresistive elements 210C in which the perpendicular magnetization film is used in the storage layer 218 are formed in the same layer (FIG. 16 and FIG. 17) and when the magnetoresistive elements 210E and the magnetoresistive elements 210C are formed in different layers (FIG. 18, FIG. 19, and FIG. 20), the magnetoresistive elements can be manufactured using a method explained below.

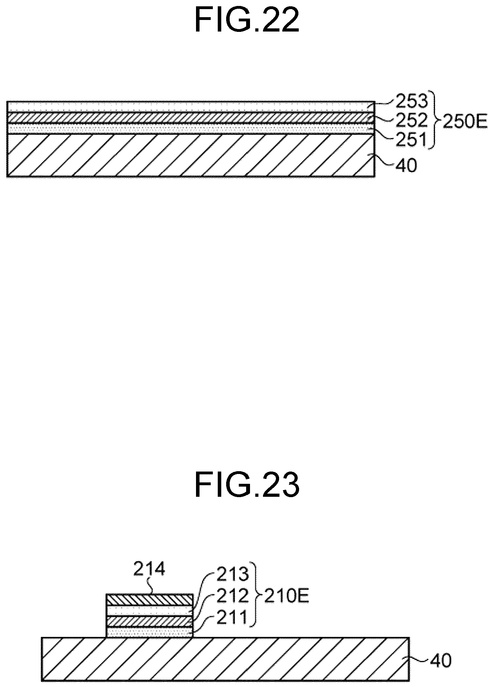

First, an example is explained in which the magnetoresistive elements 210E in which the in-plane magnetization film is used in the storage layer 213 and the magnetoresistive elements 210C in which the perpendicular magnetization film is used in the storage layer 218 are formed in the same layer. FIG. 22 to FIG. 30 are process cross-sectional views illustrating an example of a method of manufacturing magnetoresistive elements according to the present embodiment.

In this manufacturing method, as illustrated in FIG. 22, a laminated film 250E in which a first layer 251 to be processed into the magnetization fixed layer 211, a second layer 252 to be processed into the nonmagnetic layer 212, and a third layer 253 to be processed into the storage layer 213 in the magnetoresistive element 210E are laminated in this order is formed on the entire surface of a base substrate 40 including a peripheral circuit. Note that the third layer 253 may be an in-plane magnetization film.

Subsequently, as illustrated in FIG. 23, the laminated film 250E is processed into a mesa-shaped magnetoresistive element 210E using, for example, the photolithography technique and the etching technique. An upper electrode 214 is formed on the upper surface of the magnetoresistive element 10.

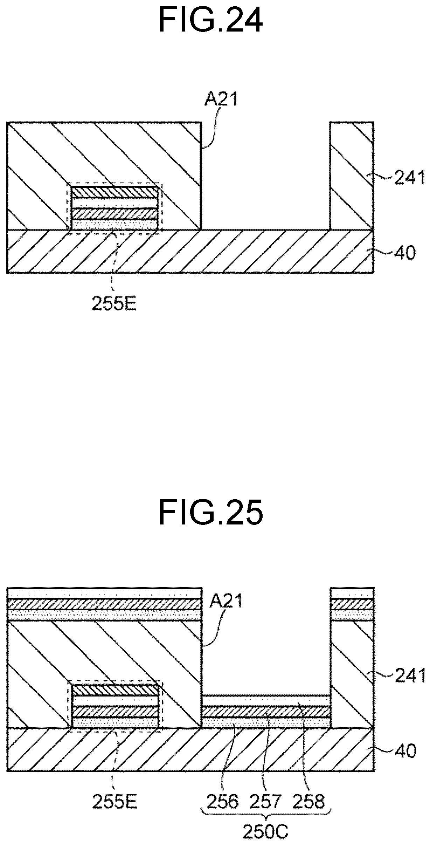

Subsequently, an insulating layer 241 is formed to embed a structure 255E including the magnetoresistive element 210E and the upper electrode 214 using, for example, the CVD (Chemical Vapor Deposition) method or the sputtering method. Subsequently, as illustrated in FIG. 24, a trench A21 for forming the magnetoresistive element 210C is formed in the formed insulating layer 241 using, for example, the photolithography technique and the etching technique. Note that the upper surface of the insulating layer 241 may be planarized by, for example, CMP (Chemical Mechanical Polishing).

Subsequently, as illustrated in FIG. 25, a laminated film 250C in which a first layer 256 to be processed into the magnetization fixed layer 216, a second layer 257 to be processed into the nonmagnetic layer 217, and a third layer 258 to be processed into the storage layer 218 in the magnetoresistive element 210C are laminated in this order is formed on the base substrate 40 exposed in the trench A21. Note that the third layer 258 may be a perpendicular magnetization film. The laminated film 250C formed on the insulating layer 241 may be removed by the lift-off method, the CMP, or the like.

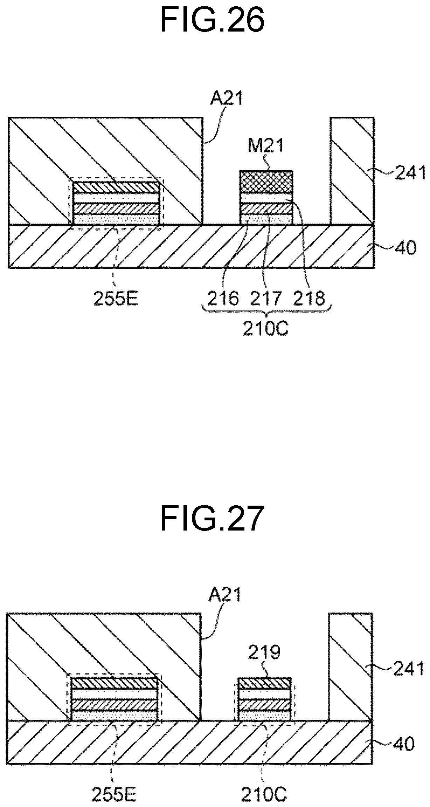

Subsequently, as illustrated in FIG. 26, a mask M21 is formed on the laminated film 250C using, for example, photolithography. The laminated film 250C exposed from the mask M21 is dug using the etching technique such as RIE (Reactive Ion Etching) to form a mesa-shaped magnetoresistive element 210C.

Subsequently, as illustrated in FIG. 27, an upper electrode 219 is formed on the upper surface of the magnetoresistive element 210C using, for example, the lift-off method.

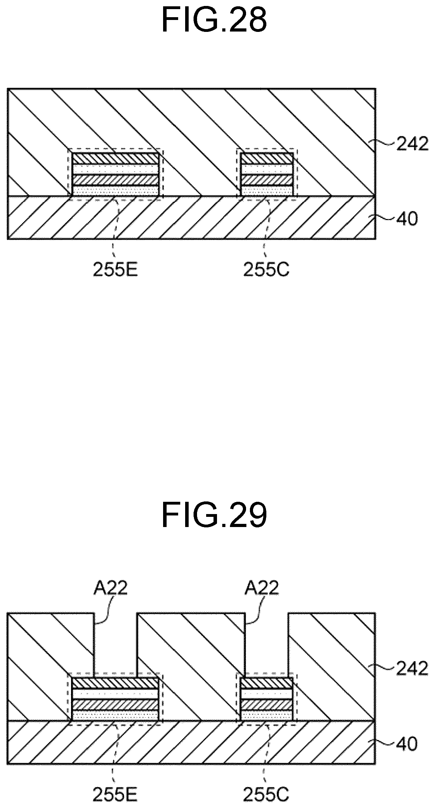

Subsequently, as illustrated in FIG. 28, the trench A21 of the insulating layer 241 is buried using, for example, the CVD method or the sputtering method to form an insulating layer 242 that covers the structure 255E including the magnetoresistive element 210E and the upper electrode 214 and a structure 255C including the magnetoresistive element 210C and the upper electrode 219. Note that the upper surface of the insulating layer 242 may be planarized by, for example, CMP.

Subsequently, as illustrated in FIG. 29, an opening A22 for exposing parts of the upper surfaces of the respective upper electrodes 214 and 219 is formed using, for example, the photolithography technique and the etching technique.

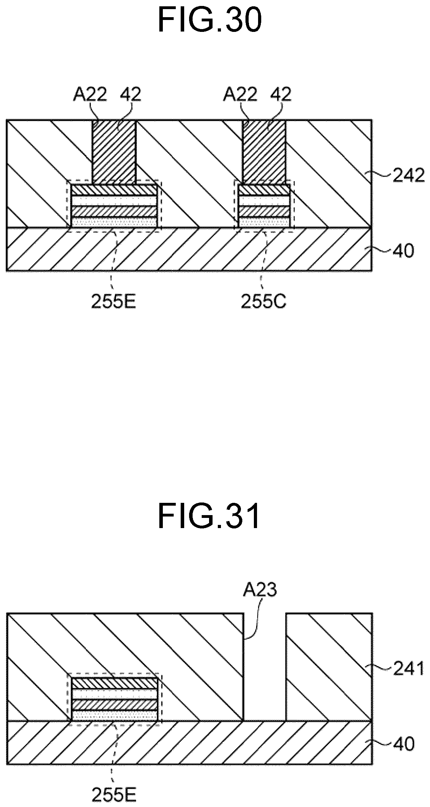

Subsequently, as illustrated in FIG. 30, a wire 42 connected to the upper electrode 214 or 219 is embedded in the opening A22. Thereafter, a wire (not illustrated) connecting the wire 42 to a power supply voltage VDD is formed on the insulating layer 242, whereby the magnetoresistive element according to the present embodiment is manufactured.

Subsequently, an example of a manufacturing method in the case in which the magnetoresistive element 210E and the magnetoresistive element 210C are formed in different layers is explained. FIG. 31 to FIG. 37 are process cross-sectional views illustrating an example of another method for manufacturing a magnetoresistive element according to the present embodiment.

First, as in the method explained above, a structure 255E including the magnetoresistive element 210E and the upper electrode 214 is formed on the base substrate 40 including the peripheral circuit.

Subsequently, for example, the insulating layer 241 is formed to embed the structure 255E including the magnetoresistive element 210E and the upper electrode 214 using the CVD method or the sputtering method. Subsequently, as illustrated in FIG. 31, a trench A23 for exposing a lower electrode in the base substrate 40 is formed in the formed insulating layer 241 using, for example, the photolithography technique and the etching technique. Note that the upper surface of the insulating layer 241 may be planarized by, for example, CMP.

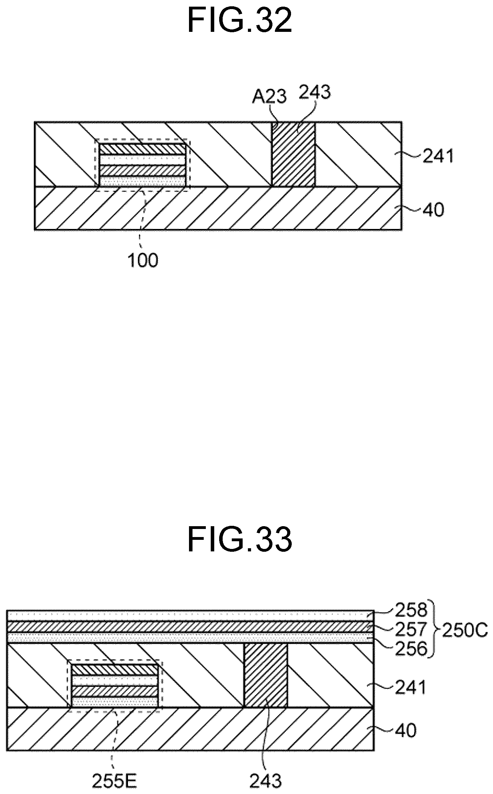

Subsequently, as illustrated in FIG. 32, a wire 243 connected to the lower electrode of the base substrate 40 is embedded in the trench A23 of the insulating layer 241.

Subsequently, as illustrated in FIG. 33, the laminated film 250C in which the first layer 256 to be processed into the magnetization fixed layer 216, the second layer 257 to be processed into the nonmagnetic layer 217, and the third layer 258 to be processed into the storage layer 218 in the magnetoresistive element 210C are laminated in this order is formed on the insulating layer 241. Note that the third layer 258 may be a perpendicular magnetization film.

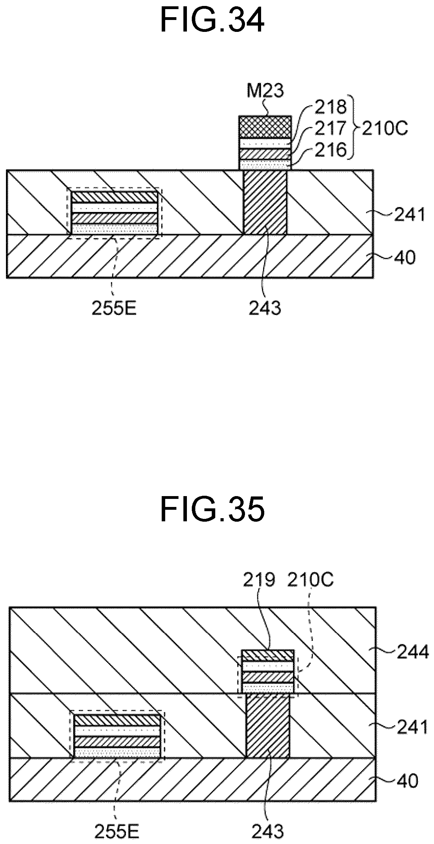

Subsequently, a mask M23 is formed on the laminated film 250C using, for example, the photolithography. The laminated film 250C exposed from the mask M23 is dug using the ng technique such as RIE to form the mesa-shaped magnetoresistive element 210C. Subsequently, as illustrated in FIG. 34, for example, the upper electrode 219 is formed on the upper surface of the magnetoresistive element 210C using, for example, the lift-off method.

Subsequently, as illustrated in FIG. 35, an insulating layer 244 is formed to embed the structure 255C including the magnetoresistive element 210C and the upper electrode 219 using, for example, the CVD method or the sputtering method. Note that the upper surface of the insulating layer 244 may be planarized by, for example, CMP.

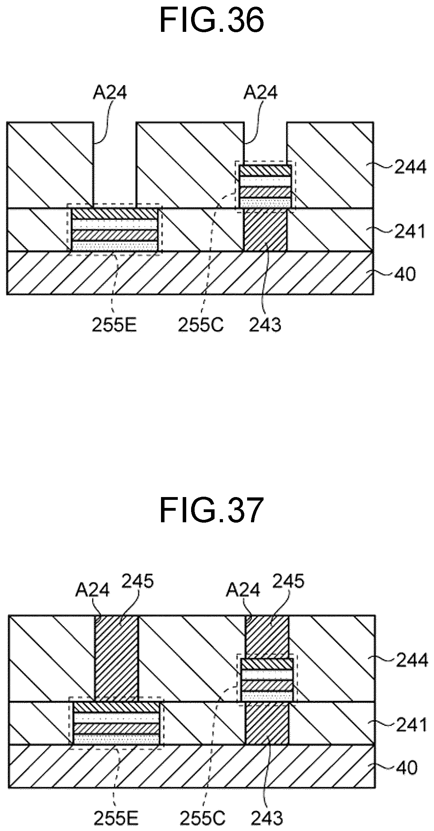

Subsequently, as illustrated in FIG. 36, an opening A24 for exposing parts of the upper surfaces of the respective upper electrodes 214 and 219 is formed using, for example, the photolithography technique and the etching technique.

Subsequently, as illustrated in FIG. 37, a wire 245 connected to the upper electrode 214 or 219 is embedded in the opening A24. Thereafter, a wire (not illustrated) for connecting the wire 245 to the power supply voltage VDD is formed on the insulating layer 244, whereby the magnetoresistive element according to the present embodiment is manufactured.

<1.5 Configuration example of magnetic detection device>

(Magnetic detection device)

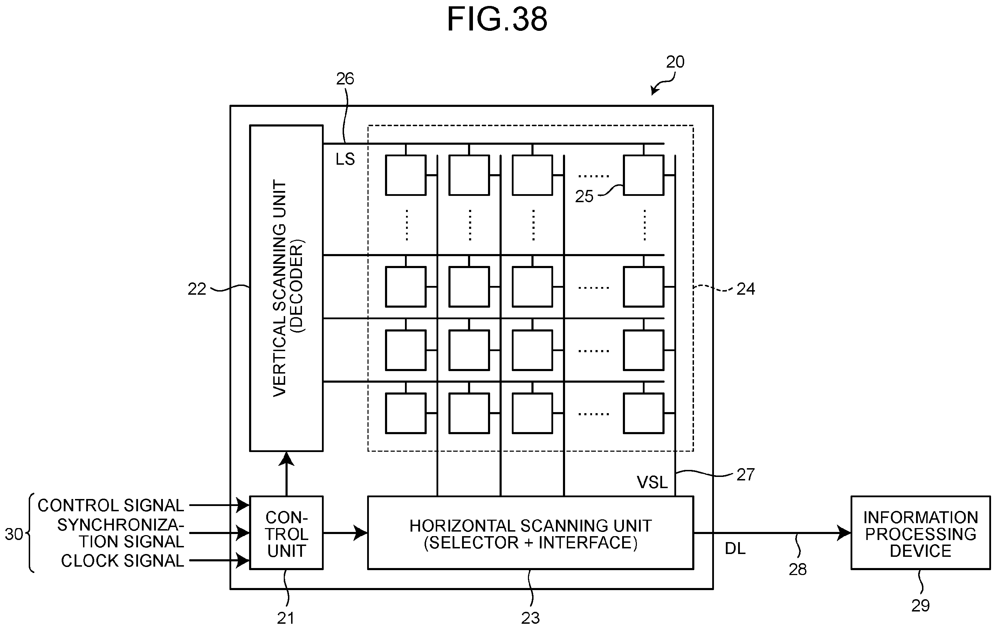

Subsequently, a configuration example of the magnetic detection device according to the present embodiment is explained with reference to FIG. 38. FIG. 38 is a block diagram illustrating a schematic configuration example of the magnetic detection device according to the present embodiment.

As illustrated in FIG. 38, the magnetic detection device 20 mainly includes a control unit 21, a vertical scanning unit (a decoder) 22, a horizontal scanning unit (a selector + an interface) 23, and a pixel array (a pixel array unit) 24.

The pixel array 24 is configured by pixel units 25 being arranged in an array of M rows and N columns. Here, M and N are 1 or 2 or more. The vertical scanning unit 22 is connected to a row selection signal line (LS) 26 extending in a row direction of the pixel array 24. The row selection signal line 26 is one or a plurality of wires for rows of the pixel array 24 and is connected to the pixel unit 25 corresponding thereto in the pixel array 24. The horizontal scanning unit 23 is connected to a vertical signal line (VSL) 27 extending in a column direction of the pixel array 24. The vertical signal line 27 is one or a plurality of wires for columns of the pixel array 24 and is connected to the pixel unit 25 corresponding thereto in the pixel array 24. The horizontal scanning unit 23 is connected to a data output line (DL) 28 in order to output detected magnetic field information as output data.

An input signal 30 of a control signal, a synchronization signal, and a clock signal is input to the control unit 21. A format of the control signal can be a format such as I2C, I3C, SPI, or GPIO. The synchronization signal may be generated on the inside of the control unit 21 instead of being input from the outside. Instead of being input from the outside, the clock signal may be generated on the inside by incorporating an oscillator in the control unit 21.

In response to the input signal 30, the control unit 21 controls the magnetic detection device 20 by outputting a row address to the vertical scanning unit 22, outputting a column address to the horizontal scanning unit 23, and outputting other necessary signals to the vertical scanning unit 22, the horizontal scanning unit 23, and the pixel unit 25.

The decoder of the vertical scanning unit 22 vertically scans the pixel array 24 and sets only a row selection signal designated by the row address input from the control unit 21 to enable.

The selector of the horizontal scanning unit 23 horizontally scans the pixel array 24, selects a specific signal designated by the column address input from the control unit 21 among vertical signals input in parallel from the pixel unit 25, and serves the selected signal for output. The interface of the horizontal scanning unit 23 converts the signal selected by the selector of the horizontal scanning unit 23 into a predetermined format (serial, parallel, compromise, or the like) and outputs the converted signal to a data output line 28.

The data output line 28 of the magnetic detection device 20 is connected to an information processing device 29 such as a computer, a smartphone, and a dedicated electronic device, whereby a magnetic field-related image based on a measurement result by the magnetic detection device 20 can be displayed. Note that the magnetic field-related image may be a magnetic field image generated from data detected by the magnetic detection device 20 or may be an image processed by any conversion operation.

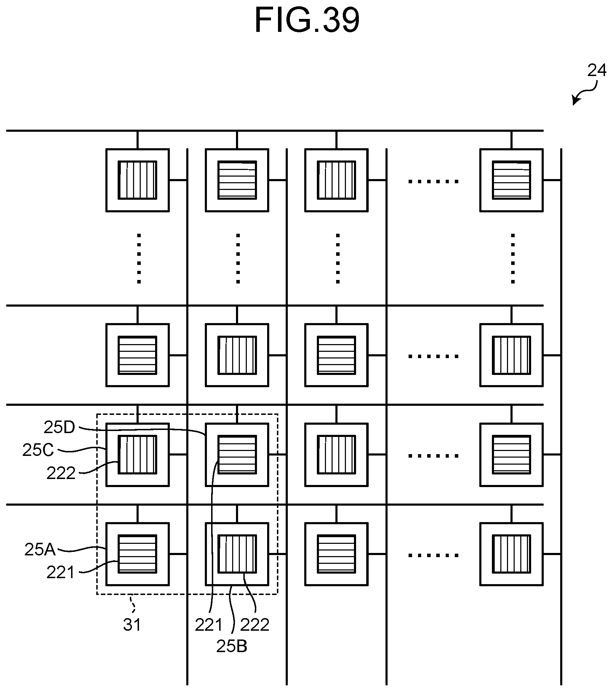

A basic array pattern 31 (see FIG. 39) including the pixel unit 25 or the plurality of pixel units 25 includes a plurality of magnetoresistive elements 10 whose resistance values change depending on the intensity and the direction of an input magnetic field. As explained above, the magnetoresistive element 10 has the easy axis to which the magnetization direction of the storage layer 13 is more easily directed than other directions and the difficult axis to which the magnetization direction of the storage layer 13 is less easily directed than the other directions. In the present embodiment, the direction of the easy axis of at least one magnetoresistive element 10 included in the pixel unit 25 or the basic array pattern 31 is different from the directions of the easy axes of the other magnetoresistive elements 10 included in the pixel unit 25 or the basic array pattern 31. Accordingly, in the present embodiment, it is possible to detect the magnitudes of the input magnetic fields B in two or more axial directions with high sensitivity at a matched position in a space.

Note that the basic array pattern 31 (see FIG. 39) including the pixel unit 25 or the plurality of pixel units 25 may include the magnetoresistive elements 10 of different types formed in different layers of a semiconductor wafer illustrated in FIG. 18 to FIG. 20.

Furthermore, the pixel unit 25 includes a reading circuit. The reading circuit outputs a reading signal to the vertical signal line 27 based on enable setting of the row selection signal line 26. The pixel unit 25 can include a read signal holding unit (not illustrated) that holds a read signal. The pixel unit 25 can include a circuit configuration including a resistor, a comparator, a transistor, and a capacitor as a circuit for reading a change in a resistance value of the magnetoresistive element 10. In the present embodiment, by using the detection circuit, it is possible to measure a time in which the magnetoresistive elements 10 are in the parallel state (a first stay time) and a time in which the magnetoresistive elements 10 are in the antiparallel state (a second stay time), that is, a time in which the magnetoresistive elements 10 are in the high resistance state and a time in which the magnetoresistive elements 10 are in the low resistance state and calculate the stay time difference S which is a difference between these times. Then, in the present embodiment, only a component along the easy axis direction of the magnetoresistive element 10 determined by the magnetization direction of the magnetization fixed layer 11 of the magnetoresistive element 10 can be detected from an input magnetic field having any angle according to the calculated stay time difference S. Furthermore, in the present embodiment, since the pixel unit 25 is linked in advance with coordinate information indicating a position on the pixel array 24, a signal output from the pixel unit 25 can be linked with the coordinate information.

Therefore, in the present embodiment, by adopting the configuration explained above, the magnetic detection device 20 can output data relating to a magnetic field measurement result in a specific direction in correlation with the coordinate information. The information processing device 29 can display a magnetic field-related image.

Furthermore, in the present embodiment, when the pixel unit 25 includes a plurality of magnetoresistive elements 10 of the same type, the detection circuit may calculate the input magnetic field based on a difference between an integrated value of a time of being in the parallel state and an integrated value of a time of being in the antiparallel state in each of the plurality of magnetoresistive elements 10.

Since the magnetization direction of the storage layer 13 is limited to the two states of the parallel state and the antiparallel state, in the present embodiment, at least one of the time in which the magnetoresistive element 10 is in the parallel state and the time in which the magnetoresistive element 10 is in the antiparallel state may be measured and the stay time difference S may be calculated based on the time.

Note that, in the present embodiment, the configuration of the magnetic detection device is not limited to the example illustrated in FIG. 38.

(Array example)

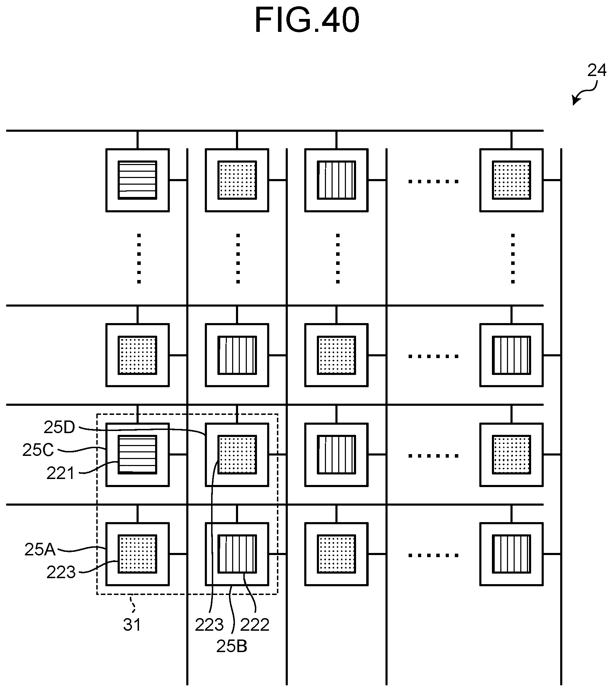

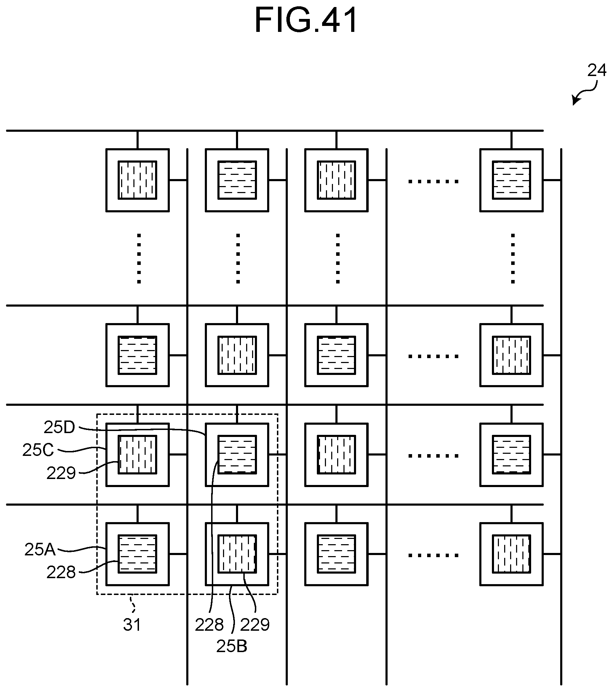

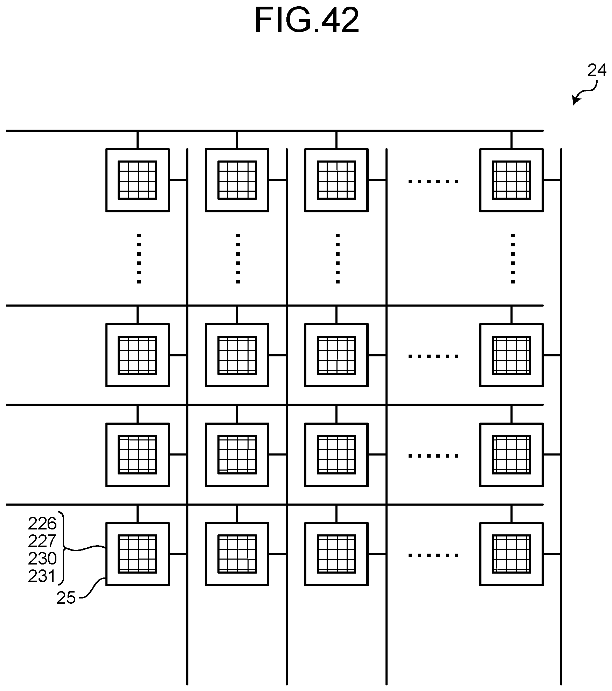

Subsequently, an array example of pixel units of the pixel array 24 is explained in detail with reference to FIG. 39 to FIG. 42. FIG. 39 is a block diagram illustrating a first example of an array of pixel units according to the present embodiment. FIG. 40 is a block diagram illustrating a second example of the array of the pixel units according to the present embodiment. FIG. 41 is a block diagram illustrating a third example of the array of the pixel units according to the present embodiment. FIG. 42 is a block diagram illustrating a fourth example of the array of the pixel units according to the present embodiment.

Note that, in the following explanation, the pixel unit 25 includes one magnetoresistive element 10 or a plurality of magnetoresistive elements 10 of the same type or different types identified as being located at predetermined one coordinate point on the pixel array 24 of the magnetic detection device 20.

FIG. 39 is a diagram illustrating the first example of the array of the pixel units according to the present embodiment. One pixel unit 25 includes an array of the magnetoresistive elements 10 of one type. In the pixel array 24, basic array patterns 31 formed by pluralities of the pixel units 25 being arrayed in an array are further arranged in an array. That is, the basic array pattern 31 can include magnetoresistive element type pixel units of a plurality of types grouped for each of the types of the magnetoresistive elements 10.

Specifically, in the example illustrated in FIG. 39, a lowermost left pixel unit 25A of the pixel array 24 includes the array 222 of the magnetoresistive elements explained above. A pixel unit 25B and a pixel unit 25C adjacent to the pixel unit 25A include the array 221 of the magnetoresistive elements explained above. A pixel unit 25D adjacent to the pixel unit 25B and the pixel unit 25C includes the array 222 of the magnetoresistive elements explained above. As explained above, in the example, the basic array pattern 31 is configured by an array of $2 \times 2$ pixel units. The remaining pixel units 25 include the array 221 of the magnetoresistive elements or the array 222 of the magnetoresistive elements obtained by repeating the basic array pattern 31. As explained above, the array 221 of the magnetoresistive elements is capable of detecting the magnitude of the input magnetic field B in the X direction with high sensitivity and the array 222 of the magnetoresistive elements is capable of detecting the magnitude of the input magnetic field B in the Y direction with high sensitivity. Therefore, the array of the pixel units illustrated in FIG. 39 is capable of implementing two-axis magnetic field detection.

FIG. 40 is a diagram illustrating a second example of the array of the pixel units according to the present embodiment. One pixel unit 25 includes an array of the magnetoresistive elements 10 of one type. In the pixel array 24, basic array patterns 31 formed by pluralities of the pixel units 25 being arrayed in an array are further arranged in an array. That is, the basic array pattern 31 can include magnetoresistive element type pixel units of a plurality of types grouped for each of the types of the magnetoresistive elements 10.

Specifically, in the example illustrated in FIG. 40, the lowermost left pixel unit 25A of the pixel array 24 includes the above-described array 223 of magnetoresistive elements, and the pixel unit 25B adjacent to the pixel unit 25A includes the above-described array 222 of magnetoresistive elements. Another pixel unit 25C adjacent to the pixel unit 25A includes the above-described array 221 of magnetoresistive elements, and the pixel unit 25B and the pixel unit 25D adjacent to the pixel unit 25C include the above-described array 223 of magnetoresistive elements. As explained above, in the example, the basic array pattern 31 is configured by an array of $2 \times 2$ pixel units. The remaining pixel units 25 include an array 221 of magnetoresistive elements, an array 222 of magnetoresistive elements, or an array 223 of magnetoresistive elements in which the basic array pattern 31 is repeated. As explained above, the array 221 of the magnetoresistive elements is capable of detecting the magnitude of the input magnetic field B in the X direction with high sensitivity and the array 222 of the magnetoresistive elements is capable of detecting the magnitude of the input magnetic field B in the Y direction with high sensitivity. Furthermore, the array 223 of magnetoresistive elements can detect the magnitude of the input magnetic field B in the Z direction with high sensitivity. Therefore, the array of pixel units illustrated in FIG. 40 can realize three-axis magnetic field detection.

FIG. 41 is a diagram illustrating a third example of the array of pixel units according to the present embodiment. One pixel unit 25 includes an array of the magnetoresistive elements 10 of one type. In the pixel array 24, basic array patterns 31 formed by pluralities of the pixel units 25 being arrayed in an array are further arranged in an array. That is, the basic array pattern 31 can include magnetoresistive element type pixel units of a plurality of types grouped for each of the types of the magnetoresistive elements 10.

Specifically, in the example illustrated in FIG. 41, the lowermost left pixel unit 25A of the pixel array 24 includes the array 228 of the magnetoresistive elements explained above and the pixel unit 25B and the pixel unit 25C adjacent to the pixel unit 25A include the array 229 of the magnetoresistive elements explained above. The pixel unit 25D adjacent to the pixel unit 25B and the pixel unit 25C includes the array 228 of the magnetoresistive elements explained above. As explained above, in the example, the basic array pattern 31 is configured by an array of $2 \times 2$ pixel units. The remaining pixel units 25 include the array 228 of the magnetoresistive elements or the array 229 of the magnetoresistive elements obtained by repeating the basic array pattern 31. As explained above, the array 228 of the magnetoresistive elements is capable of detecting the magnitude and the direction of the input magnetic field B in the XZ in-plane direction with high sensitivity and the array 229 of the magnetoresistive elements is capable of detecting the magnitude of the input magnetic field B in the YZ in-plane direction with high sensitivity. Therefore, the array of the pixel units illustrated in FIG. 41 is capable of implementing three-axis magnetic field detection.

FIG. 42 is a diagram illustrating a fourth example of the array of the pixel units according to the present embodiment. The pixel array 24 includes the pixel unit 25 including an array of the magnetoresistive elements 10 of one type. In the example illustrated in FIG. 42, the array of the magnetoresistive elements 10 can be selected from the array 226 of the magnetoresistive elements, the array 227 of the magnetoresistive elements, the array 230 of the magnetoresistive elements, and the array 231 of the magnetoresistive elements explained above. When the array 226 of the magnetoresistive elements is selected as the array of the magnetoresistive elements, the array of the pixel units illustrated in FIG. 42 can implement two-axis magnetic field detection. When the array of the magnetoresistive elements other than the array 226 is selected, the array of the pixel units illustrated in FIG. 42 can implement three-axis magnetic field detection. By taking the array of the pixel units illustrated in FIG. 42, the magnetic detection device 20 can improve the resolution of a magnetic field-related image.

Note that, in the present embodiment, the array of the pixel units of the pixel array 24 is not limited to the examples illustrated in FIG. 39 to FIG. 42.

<1.6 Operation examples of magnetic detection device>

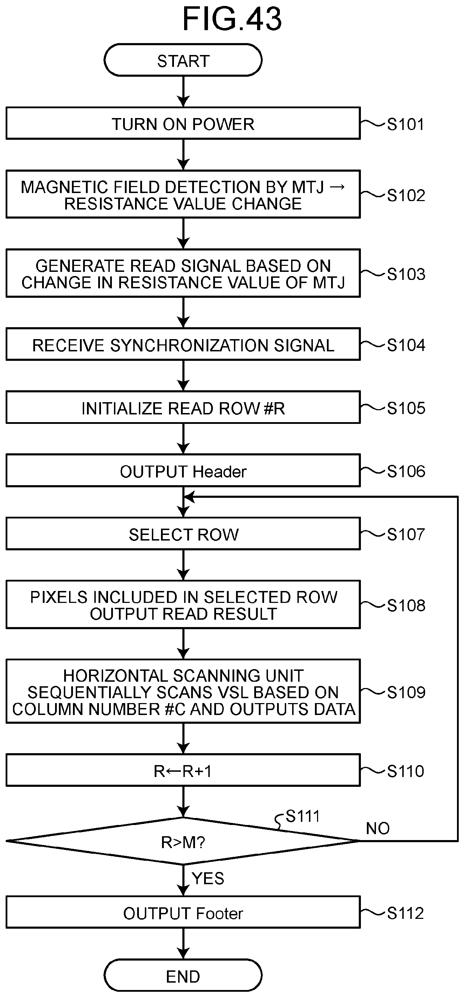

Subsequently, operation examples of the magnetic detection device 20 according to the present embodiment is explained with reference to FIG. 43 to FIG. 46.

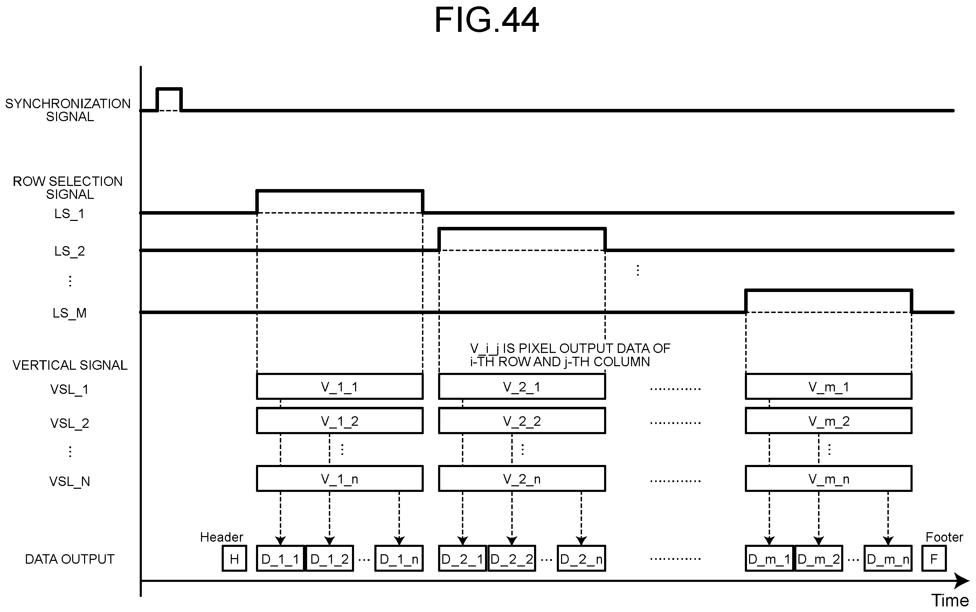

FIG. 43 and FIG. 44 are respectively a flowchart and a timing diagram illustrating a first operation example of the magnetic detection device 20 according to the present embodiment. The operation example is equivalent to a case in which magnetic field detection by the magnetoresistive element 10 is performed as needed at a point in time of scanning explained below. Note that, in the operation example, as illustrated in FIG. 44, various control signals are generated with a clock signal set as a time reference and a selection signal for selecting a row to be read is generated based on a control signal.

As illustrated in FIG. 43, first, power is turned on (Step S101). When the power is turned on, since electric power is supplied to the pixel unit 25 and the magnetoresistive element (MTJ) 10 included in the pixel unit 25 detects an input magnetic field, a resistance value of the magnetoresistive element 10 changes (Step S102). Subsequently, based on the resistance value change of the magnetoresistive element 10, a read signal is generated by the reading circuit included in the pixel unit 25 (Step S103).

Subsequently, as illustrated in FIG. 43, when the magnetic detection device 20 receives a synchronization signal (Step S104), a read row is initialized and a counter R is set to 1 (Step S105). Then, a header (H) meaning the head of the reading is output (Step S106). A row designated by the counter R is selected by the vertical scanning unit 22 and a selected row selection signal line 26 is enabled (Step S107).

Subsequently, as illustrated in FIG. 44, read signals that are read results of the pixel units 25 connected to the selected row selection signal line 26 are output to vertical signals VSL_1 to VSL_N as vertical signals V_R_1 to V_R_N (Step S108). Here, R is a value of the counter R and V_i_j is read data of the pixel unit 25 of an i-th row and a j-th column.

Subsequently, as illustrated in FIG. 44, the horizontal scanning unit 23 sequentially outputs a vertical signal V_R_C to a data line DL as output data D_R_C based on a column counter C. Here, C is a value of the counter C and D_i_j is output data of the pixel unit 25 of the i-th row and the j-th column. At this time, the horizontal scanning unit 23 may convert a vertical signal into output data according to any conversion operation with an image output interface unit. This data output is performed for all the pixel units 25 connected to the selected row selection signal line 26 by the horizontal scanning unit 23 scanning the column counter C (Step S109).

Subsequently, the counter R is incremented (Step S110) and the value of the counter R is compared with a row number M (Step S111). In the case of R > M (Step S111; Yes), a footer meaning the end of the reading is output and the reading ends (Step S112). In the case of R ≤ M (Step S111; No), the processing returns to the processing in Step S107 in order to read the next row.

As explained above, output data of all the pixel units 25 are output. Note that a method of generating a selection signal may be changed based on a control signal received by the magnetic detection device 20. For example, various variations such as scanning only any one axis of the X axis, the Y axis, and the Z axis or scanning the X axis, the Y axis, and the Z axis in order can be adopted.

In the first operation example of the magnetic detection device 20 according to the present embodiment illustrated in FIG. 43 and FIG. 44, a read signal from the two-dimensionally arranged pixel units 25 is scanned based on the synchronization signal and is output in correlation with coordinate information. Accordingly, the information processing device 29 is capable of performing information processing, for example, image display.

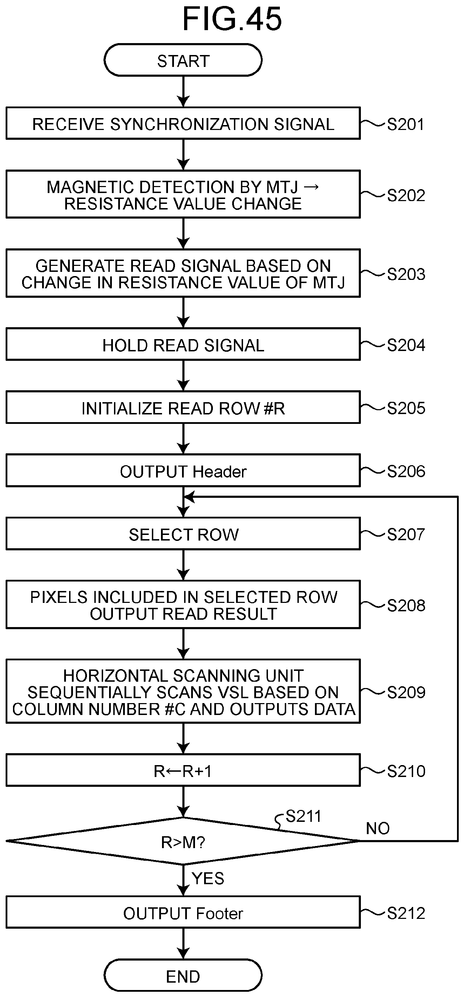

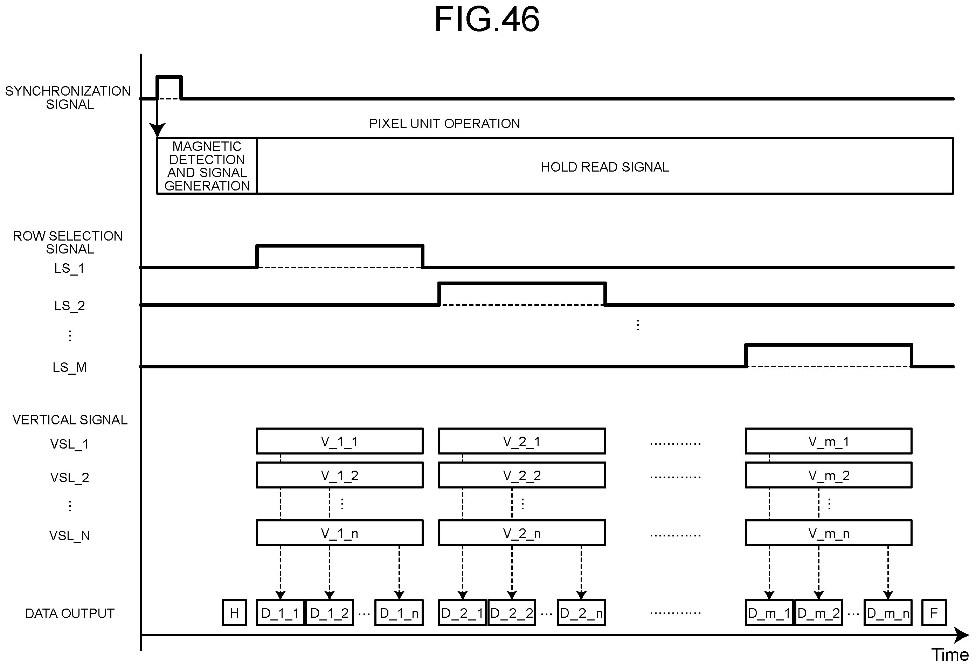

FIG. 45 and FIG. 46 are respectively a flowchart and a timing diagram illustrating a second operation example of the magnetic detection device 20 according to the present embodiment. The operation example is equivalent to a case in which magnetic field detection by a plurality of pixel units is simultaneously performed at predetermined timing. In the flowchart of FIG. 45, it is assumed that the power is turned on first.

First, as illustrated in FIG. 45, the magnetic detection device 20 receives a synchronization signal (Step S201). Subsequently, operations in Step S202 and Step S203 are performed. However, since the steps are the same as Step S102 and Step S103 in FIG. 43 explained above, explanation of the steps is omitted here. Subsequently, a read signal generated in the pixel unit 25 is held in a read signal holding unit (not illustrated) included in the pixel unit 25 (Step S204).

Subsequently, operations in Step S205 and Step S206 are performed. However, since the steps are the same as Step S105 and Step S106 in FIG. 43 explained above, explanation of the steps is omitted here. Subsequently, a row designated by the counter R is selected by the vertical scanning unit 22 and the selected row selection signal line 26 is enabled (Step S207).

As illustrated in FIG. 46, read signals held in the read signal holding units of the pixel units 25 connected to the selected row selection signal line 26 are output to the vertical signals VSL_1 to VSL N as the vertical signals V_R 1 to V_R N (Step S208). Here, R is a value of the counter R and V_i_j is read data of the pixel unit 25 of an i-th row and a j-th column.

Subsequently, as illustrated in FIG. 46, the horizontal scanning unit 23 sequentially outputs the vertical signal V_R_C to the data line DL as the output data D_R_C based on the column counter C. Here, C is a value of the counter C and D_i_j is output data of the pixel unit 25 of the i-th row and the j-th column. At this time, the horizontal scanning unit 23 may convert a vertical signal into output data according to any conversion operation with an image output interface unit. This data output is performed for all the pixel units 25 connected to the selected row selection signal line 26 by the horizontal scanning unit 23 scanning the column counter C (Step S209).

Subsequently, the counter R is incremented (Step S210) and the value of the counter R is compared with the row number M (Step S211). In the case of R > M (Step S211; Yes), a footer meaning the end of the reading is output and the reading ends (Step S212). In the case of R ≤ M (Step S211; No), the processing returns to Step S207 in order to read the next row.

As explained above, output data of all the pixel units 25 are output. Note that a method of generating a selection signal may be changed based on a control signal received by the magnetic detection device 20. For example, various variations such as scanning only any one axis of the X axis, the Y axis, and the Z axis or scanning the X axis, the Y axis, and the Z axis in order can be adopted.

According to the second operation example of the magnetic detection device 20 according to the present embodiment illustrated in FIG. 45 and FIG. 46, in addition to the action and the effects of the first operation example explained above, it is possible to secure the simultaneity of the measured magnetic field by holding the read signal from the two-dimensionally arranged pixel units 25 based on the synchronization signal.

Note that the present embodiment is not limited to the operations of the magnetic detection device 20 as illustrated in FIG. 43 to FIG. 46.

<1.7 Output data format examples>

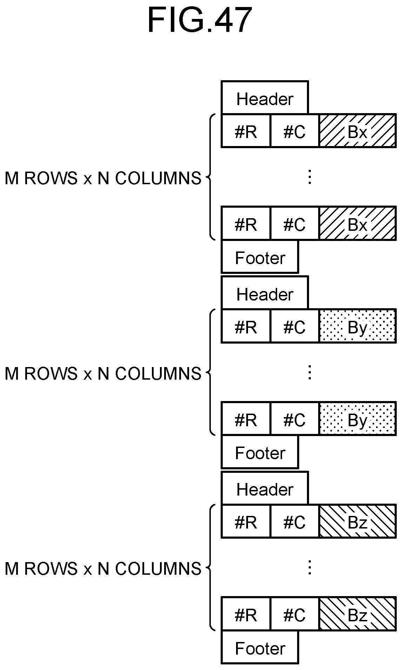

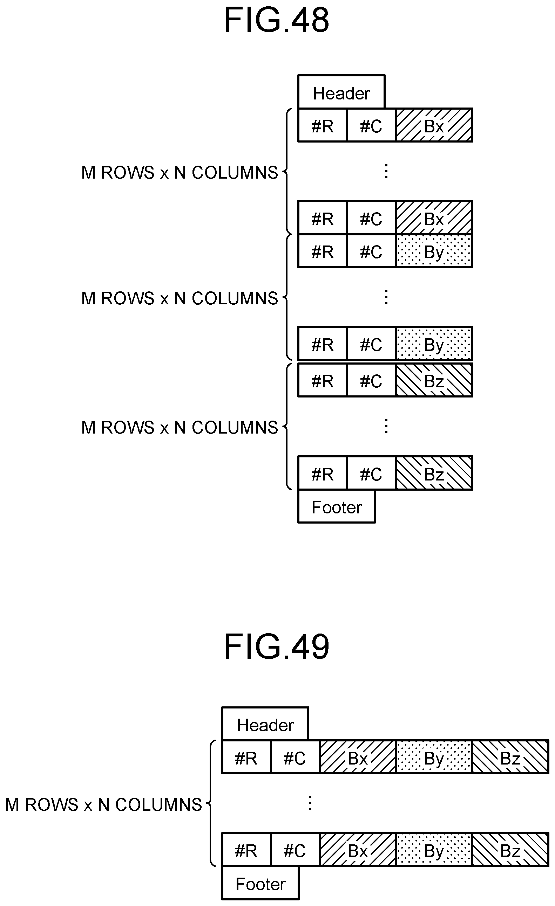

Subsequently, output data format examples of the magnetic detection device 20 according to the present embodiment are explained with reference to FIG. 47 to FIG. 49. FIG. 47 is a first output data format example of the magnetic detection device 20 according to the present embodiment. FIG. 48 is a second output data format example of the magnetic detection device 20 according to the present embodiment. FIG. 49 is a third output data format example of the magnetic detection device 20 according to the present embodiment. In FIGS. 47 to 49, #R represents a row number, #C represents a row number, Bx represents an X-axis component of a magnetic field, By represents a Y-axis component of the magnetic field, and Bz represents a Z-axis component of the magnetic field.

FIG. 47 is a first output data format example of the magnetic detection device 20 according to the present embodiment. In the first output data format example, three read operations are continuously performed in order to detect three-axis magnetic field components. Accordingly, it is possible to transmit magnetic field measurement data for each axis to the information processing device 29.

FIG. 48 is a second output data format example of the magnetic detection device 20 according to the present embodiment. Unlike the example illustrated in FIG. 47, in the second output data format example, one read operation is performed in order to detect three-axis magnetic field components. Accordingly, it is possible to transmit the magnetic field measurement data for each axis to the information processing device 29.

FIG. 49 illustrates a third output data format example of the magnetic detection device 20 according to the present embodiment. Unlike the examples illustrated in FIG. 47 and FIG. 48, in the third output data format example, three-axis magnetic field components are collectively output. Accordingly, when reading focusing on the simultaneity of the X axis, the Y axis, and the Z axis is performed, since it is unnecessary to accumulate data for one frame, memory hardware can be saved.

Note that, in the output data format examples illustrated in FIG. 47 to FIG. 49, both or one of the row number #R and the row number #C can be omitted if the information processing device 29 allows the omission.

Note that the present embodiment is not limited to the output data format examples illustrated in FIG. 47 to FIG. 49.

<1.8 Circuit configuration examples of pixel unit>

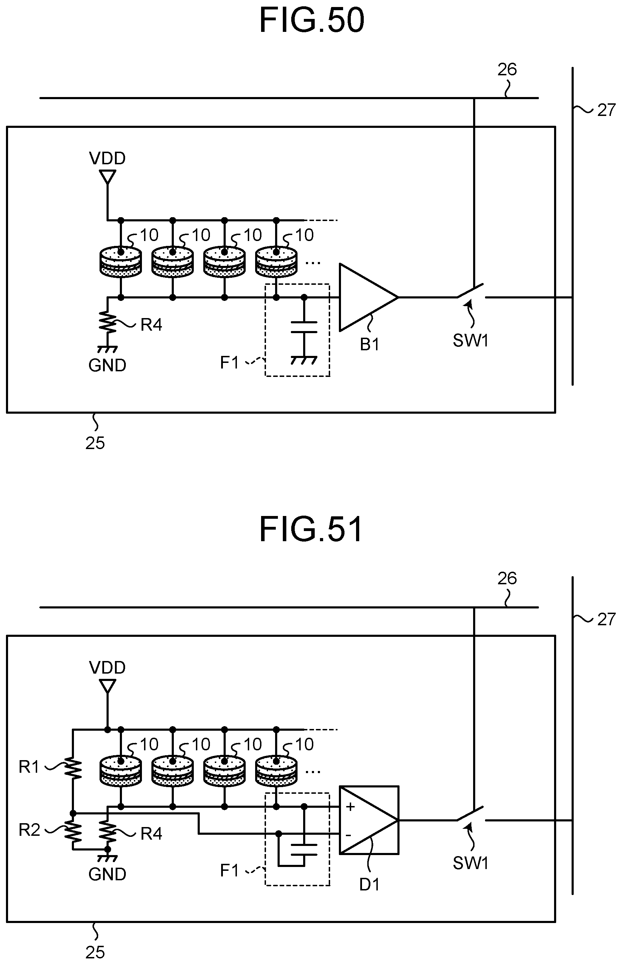

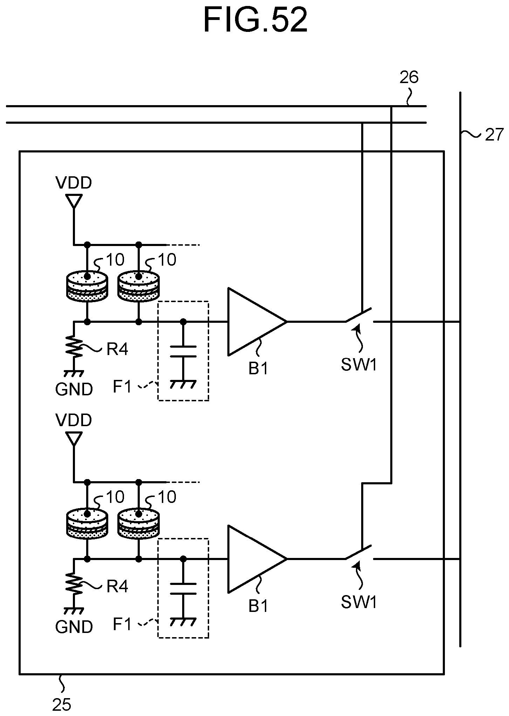

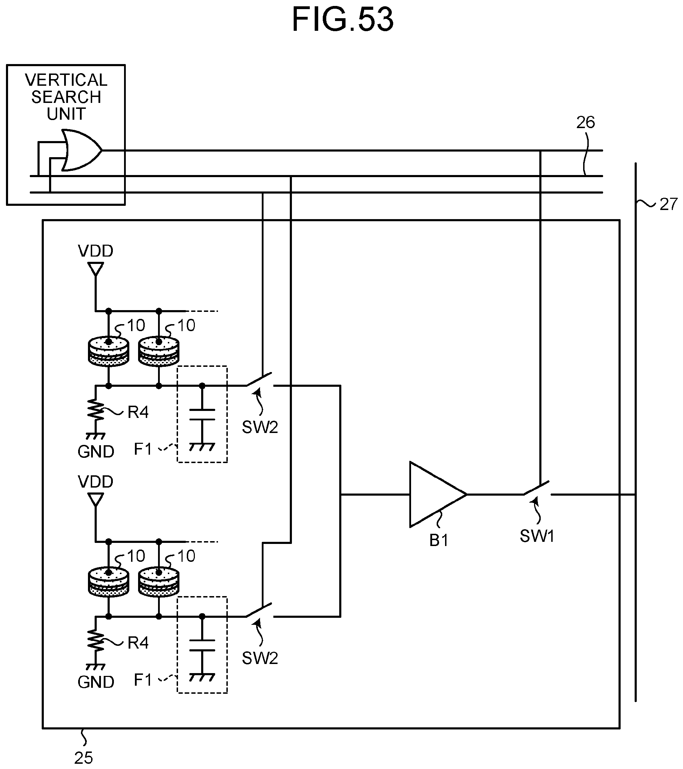

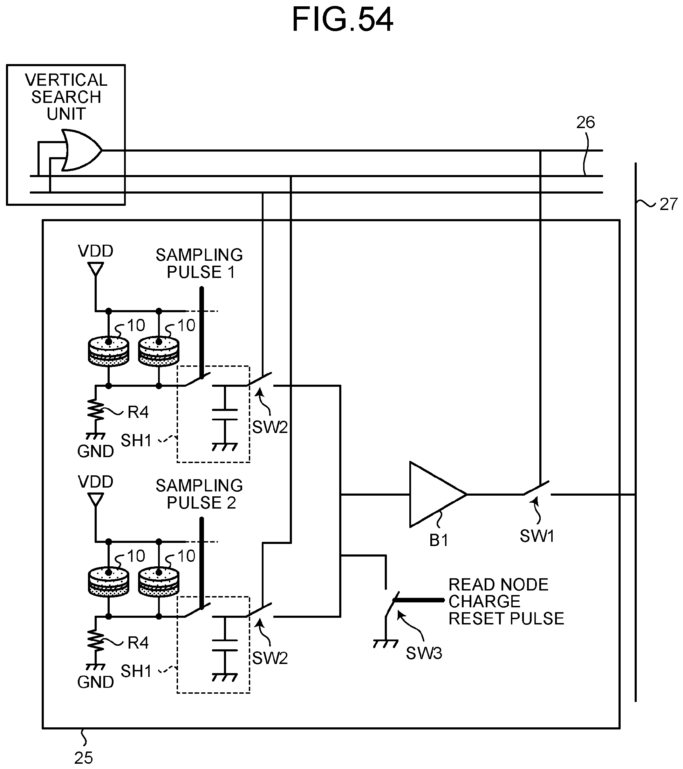

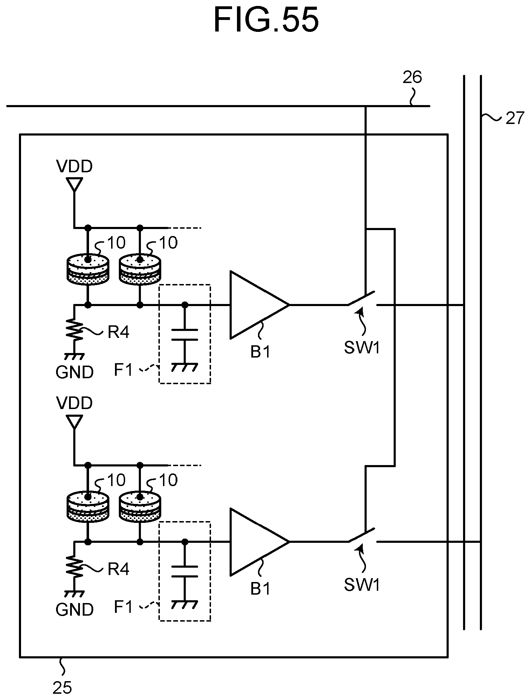

Subsequently, circuit configuration examples of the pixel unit of the magnetic detection device 20 according to the present embodiment are explained with reference to FIG. 50 to FIG. 55. FIG. 50 is a first circuit configuration example of the pixel unit 25 according to the present embodiment. FIG. 51 is a second circuit configuration example of the pixel unit 25 according to the present embodiment. FIG. 52 is a third circuit configuration example of the pixel unit 25 according to the present embodiment. FIG. 53 is a fourth circuit configuration example of the pixel unit 25 according to the present embodiment. FIG. 54 is a fifth circuit configuration example of the pixel unit 25 according to the present embodiment. FIG. 55 is a sixth circuit configuration example of the pixel unit 25 according to the present embodiment.

FIG. 50 and FIG. 51 are the first and second circuit configuration examples of the pixel unit 25 according to the present embodiment. In the pixel unit 25 in FIG. 50 and FIG. 51, all of the magnetoresistive elements 10 are connected in parallel. Therefore, the first and second circuit configuration examples in FIG. 50 and FIG. 51 are suitable when the array of the magnetoresistive elements 10 included in the pixel unit 25 includes the magnetoresistive elements 10 of one type. Note that, in the pixel unit 25 in FIG. 50 and FIG. 51, the magnetoresistive elements 10 are illustrated as being connected in parallel. However, this circuit configuration is not limited to this. For example, all of the magnetoresistive elements 10 of the pixel unit 25 may be connected in series or groups configured by pluralities of magnetoresistive elements 10 being connected in series may be connected in parallel. Then, the detection circuit explained above calculates an input magnetic field based on a resistance value of such an array of the magnetoresistive elements 10.

In FIG. 50, the magnetoresistive elements 10 connected in parallel and biasing means are connected in series to cause a power supply voltage and a ground voltage to communicate. Here, as the biasing means, for example, a series resistor R4 can be used. In FIG. 50, a voltage (a read signal) generated by the magnetoresistive elements 10 connected in parallel and the biasing means is output to the vertical signal line 27 via a buffer B1 and a row selection switch SW1. In the circuit configuration example of the pixel unit 25 in FIG. 50, filter means (for example, a capacitor)F1 may be added. In FIG. 50, the buffer B1 is provided for the purpose of driving the vertical signal line 27. Note that, in the present specification, the buffer B1 is a circuit configured to be able to drive a predetermined load and means a circuit having a gain of approximately 1.

According to the circuit configuration example of the pixel unit 25 in FIG. 50, it is possible to output a read signal of a selected pixel unit 25. In the circuit configuration in FIG. 50, even in a state before the filter means F1 is added, a filter is configured in a pseudo manner by the resistance of the magnetoresistive element 10 itself, the biasing means (the resistor R4), the parasitic capacitance of a wiring, and the input capacitance of the buffer B1. Therefore, the read signal is smoothed by such a pseudo filter. Furthermore, by adding the filter means F1, smoothing capability of the read signal can be further improved.

In FIG. 51, in addition to the circuit of FIG. 50, reference voltage generation means is further provided. The reference voltage generation means has a bridge configuration by resistors R1, R2, and R4. Further, in FIG. 51, a differential amplifier circuit D1 is provided instead of the buffer B1. Note that, in the present specification, the differential amplifier circuit D1 means a circuit that receives two input signals and amplifies the difference between the two input signals.

According to the circuit configuration example of the pixel unit 25 in FIG. 51, noise due to fluctuation of the power supply voltage VDD is canceled by amplifying and transmitting the difference between the read signal and the reference voltage. Therefore, the influence of the noise superimposed on the read signal can be reduced.

FIG. 52 to FIG. 55 are third to sixth circuit configuration examples of the pixel unit 25 according to the present embodiment. In the pixel unit 25 in FIG. 52 to FIG. 55, the magnetoresistive elements 10 are divided into a plurality of groups and the magnetoresistive elements 10 belonging to the respective groups are connected in parallel. Therefore, the circuit configuration examples illustrated in FIG. 52 to FIG. 55 are suitable when the array of the magnetoresistive elements 10 included in the pixel unit 25 includes the magnetoresistive elements 10 of two or more types. However, the magnetoresistive elements 10 of the same type may be divided into a plurality of groups.

In FIG. 52, the same circuit configuration as the circuit configuration in FIG. 50 is provided for each group of the magnetoresistive elements 10. However, the same circuit configuration as the circuit configuration in FIG. 51 may be provided for each group of the magnetoresistive elements 10. In FIG. 52, the row selection signal lines 26 are provided by a number equal to the number of groups of the magnetoresistive elements 10. In the case of such a configuration, the detection circuit explained above calculates, for each group, an input magnetic field from a resistance value of the magnetoresistive elements 10. Then, the circuit configuration in FIG. 52 is configured to output, to the vertical signal line 27, a read signal generated from a group of a predetermined part of the magnetoresistive elements 10 of the pixel unit 25 corresponding to one row selection signal line 26.

According to the circuit configuration example of the pixel unit 25 in FIG. 52, in the case of the pixel unit 25 including only the magnetoresistive elements 10 of the same type, there is an effect of reducing power consumption by partially stopping the magnetoresistive elements 10. On the other hand, in the case of the pixel unit 25 including the magnetoresistive elements 10 of a plurality of types, according to the circuit configuration example of the pixel unit 25 in FIG. 52, a magnetic field detection signal can be output for each direction.

In FIG. 53, the same circuit configuration as the circuit configuration in FIG. 50 is provided for each group of the magnetoresistive elements 10. However, the same circuit configuration as the circuit configuration in FIG. 51 may be provided for each group of the magnetoresistive elements 10. Unlike FIG. 52, in FIG. 53, selection means including a magnetoresistive element selection switch SW2 connected to the magnetoresistive elements 10 is provided before the buffer B1. In FIG. 53, a row selection switch SW1 configured to be turned on based on a logical sum of a plurality of different row selection signals is provided between the buffer B1 and the vertical signal line 27.

According to the circuit configuration example of the pixel unit 25 in FIG. 53, since the number of buffers B1 is one as compared with the circuit configuration example of the pixel unit 25 in FIG. 52, a circuit layout area and power consumption can be reduced.

In FIG. 54, a circuit configuration is the same as the circuit configuration in FIG. 53. However, in addition, sample-and-hold (S/H) means SH1 that holds a signal and a sampling hold pre-read reset switch SW3 are provided for each group of the magnetoresistive elements 10 and measurement timing can be determined from the outside. A sampling pulse for determining the measurement timing is given for each group of the magnetoresistive elements 10.

According to the circuit configuration example of the pixel unit 25 in FIG. 54, if the sampling pulse is the same among the groups of the magnetoresistive elements 10, the simultaneity of the measurement is secured and, if the sampling pulse is different among the groups of the magnetoresistive elements 10, there is an effect that the time-series data can be acquired. Since the circuit configuration example of the pixel unit 25 in FIG. 54 has a function of holding read data, the circuit configuration example is suitable for the second operation example of the magnetic detection device 20 according to the present embodiment illustrated in FIG. 45 and FIG. 46.

In FIG. 55, a circuit configuration is the same as the circuit configuration in FIG. 52. However, instead of the row selection signal lines 26 being provided by a number equal to the number of groups of magnetoresistive elements 10, the vertical signal lines 27 being provided by a number equal to the number of groups of magnetoresistive elements 10. Then, in FIG. 55, one vertical signal line 27 is connected to a group of a predetermined part of the magnetoresistive elements 10 of the pixel unit 25.

According to the circuit configuration example of the pixel unit 25 in FIG. 55, in the case of the pixel unit 25 including the magnetoresistive elements 10 of a plurality of types, there is an effect that speed can be increased by parallel reading while ensuring the simultaneity of measurement.

Note that, in the present embodiment, the circuit configuration of the pixel unit 25 of the magnetic detection device 20 is not limited to the examples illustrated in FIG. 50 to FIG. 55.

<1.9 Digitizing means examples>

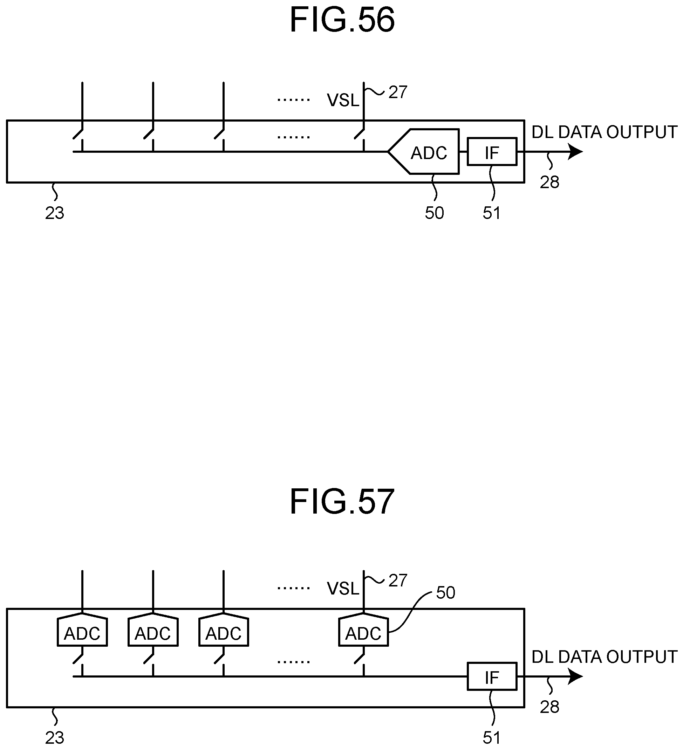

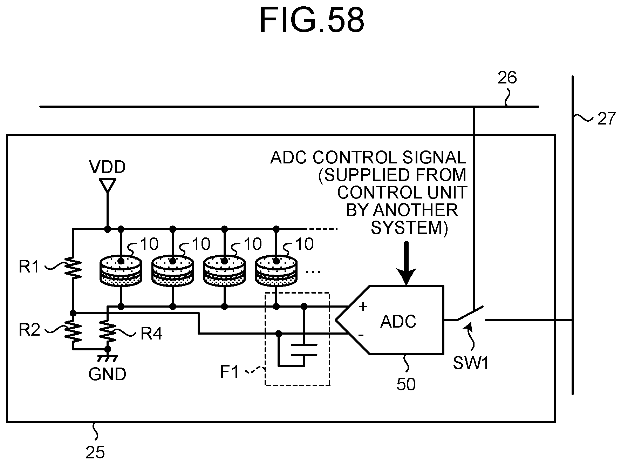

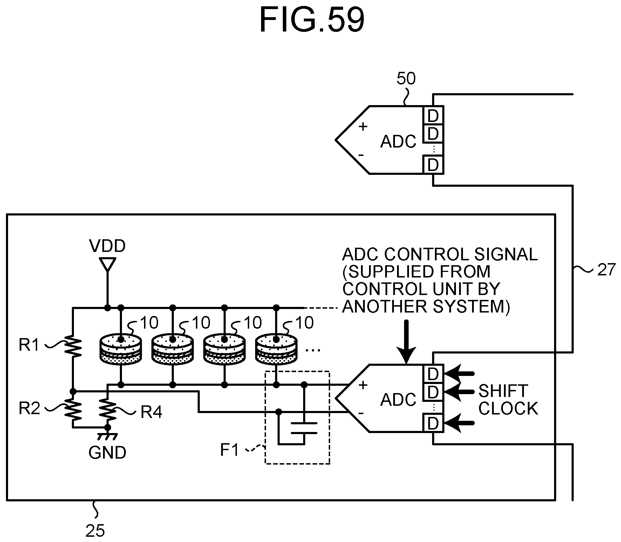

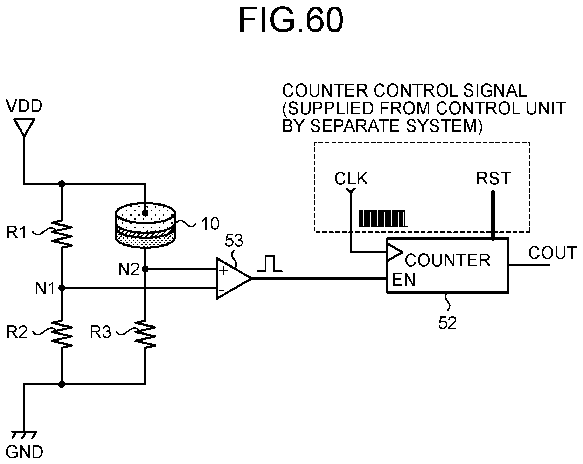

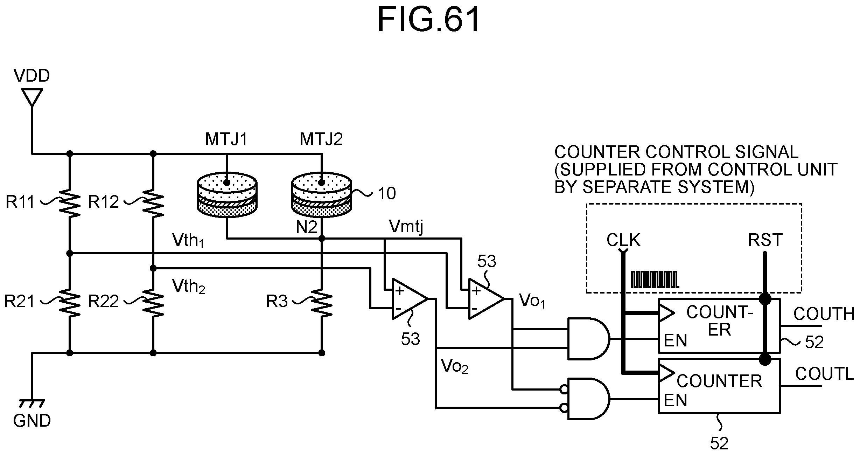

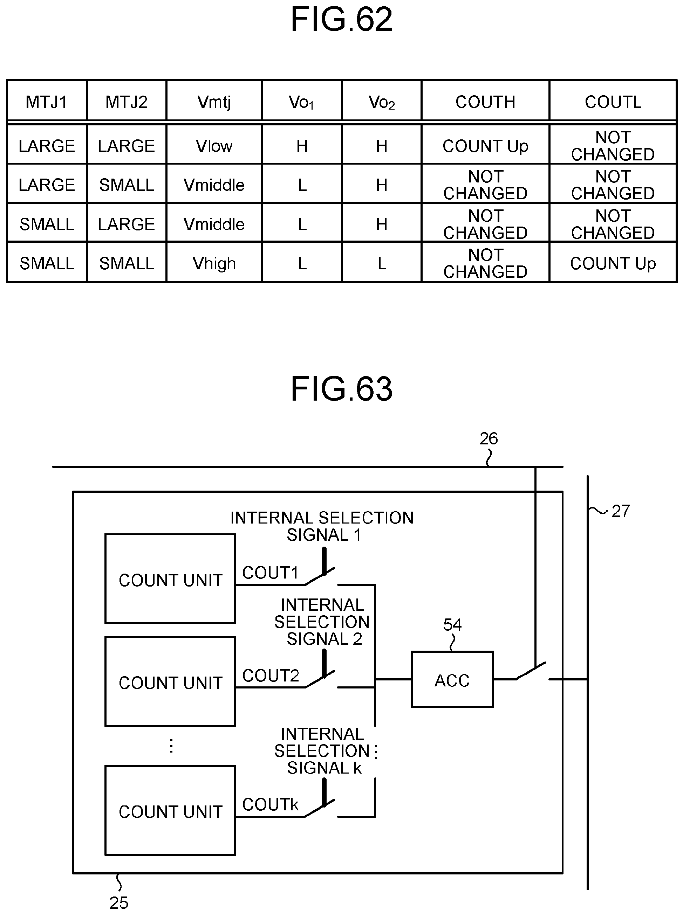

Subsequently, a circuit configuration example of the digitizing means according to the present embodiment is explained with reference to FIG. 56 to FIG. 63. FIG. 56 is a first circuit configuration example of the digitizing means according to the present embodiment. FIG. 57 is a second circuit configuration example of the digitizing means according to the present embodiment. FIG. 58 is a third circuit configuration example of the digitizing means according to the present embodiment. FIG. 59 is a fourth circuit configuration example of the digitizing means according to the present embodiment. FIG. 60 is a fifth circuit configuration example of the digitizing means according to the present embodiment. FIG. 61 is a sixth circuit configuration example of the digitizing means according to the present embodiment. FIG. 62 is an operation example of the sixth circuit configuration example. Furthermore, FIG. 63 is a seventh circuit configuration example of the digitizing means according to the present embodiment.

FIG. 56 is the first circuit configuration example of the digitizing means according to the present embodiment. In FIG. 56, the horizontal scanning unit 23 includes one analog/digital converter (ADC) (a conversion unit) 50. In the configuration example, after the horizontal scanning unit 23 selects one out of a plurality of vertical signal lines 27, an analog signal of the vertical signal line 27 is converted into a digital signal (a digital value) by the ADC 50 and output to the data output line 28 through the interface 51.

According to the digitizing means of FIG. 56, the pixel unit 25 can extract an output of a measured input magnetic field as a digital signal.

FIG. 57 is the second circuit configuration example of the digitizing means according to the present embodiment. In FIG. 57, the horizontal scanning unit 23 includes analog/digital converters (ADC) 50 equal in number to the plurality of vertical signal lines 27. In this configuration example, analog signals transmitted from the pixel units 25 via the plurality of vertical signal lines 27 are converted into digital signals in parallel by a plurality of ADCs 50 and output to the data output line 28 via the interface 51 in order from selected one.

According to the digitizing means in FIG. 57, a frame rate of an output image is improved by multi-parallelization of the ADCs 50.

FIG. 58 is the third circuit configuration example of the digitizing means according to the present embodiment. In FIG. 58, the pixel unit 25 includes one ADC 50 and converts an analog read signal into a digital read signal in the pixel unit 25. Then, the converted signal is output to the vertical signal line 27 having a bus width corresponding to the resolution of the ADC 50.

With the digitizing means in FIG. 58, since long-distance wiring for the output of the pixel unit 25 can be digital transmission, noise superimposed in analog signal transmission can be reduced.

FIG. 59 is the fourth circuit configuration example of the digitizing means according to the present embodiment. In FIG. 59, data holding units (latches) of the ADCs 50 are connected in the vertical direction to configure a shift register. Then, in the configuration example, a plurality of bits of the plurality of ADCs 50 are serially output to the vertical signal line 27 using a shift clock.

With the digitizing means in FIG. 59, since the row selection signal line 26 is unnecessary and the vertical signal may be 1 bit (since there may be one vertical signal line 27), a circuit layout area can be reduced.

In the examples in FIG. 58 and FIG. 59, the differential configuration illustrated in FIG. 51 is used. However, the single-phase configuration illustrated in FIG. 50 may be used. As illustrated in FIG. 52 to FIG. 55, the magnetoresistive elements 10 may be divided into a plurality of groups. Furthermore, in the examples in FIG. 58 and FIG. 59, the ADC 50 may be provided for each group and a read signal may be input or the read signal may be input to the ADC 50 after the group is selected.

FIG. 60 is the fifth circuit configuration example of the digitizing means according to the present embodiment. In FIG. 60, the digitizing means is configured by a count unit including one counter 52 for one magnetoresistive element 10. A comparator 53 outputs a comparison result between an output of the magnetoresistive element 10, that is, a resistance value of the magnetoresistive element 10 and a reference resistance value. Here, for example, it is assumed that the comparator 53 outputs an enable signal only when the state of the magnetoresistive element 10 is the parallel state. The counter 52 receives a clock signal as an input in addition to the output of the comparator 53. Then, the counter 52 counts the clock signal only when the output of the comparator 53 is enabled. Note that, in the circuit configuration example illustrated in FIG. 60, it is preferable that the filter means F1 connected to the magnetoresistive element 10 is not provided.

With the digitizing means in FIG. 60, since a state difference of the magnetoresistive element 10 can be directly measured by the clock signal and the counter, noise superimposed on the analog signal can be further reduced.

FIG. 61 is the sixth circuit configuration example of the digitizing means according to the present embodiment. In FIG. 61, the digitizing means is configured by a count unit including two counters 52 for two magnetoresistive elements 10. Note that, in the examples in FIG. 61 and FIG. 62, it is assumed that, when two magnetoresistive elements 10 take different states, since resistance values of the two magnetoresistive elements 10 cancel each other out, a voltage Vmtj due to the resistance values of the two magnetoresistive elements 10 becomes Vmiddle. Furthermore, in the example illustrated in FIG. 61, the counter 52 is assumed to be a circuit that counts when the two magnetoresistive elements 10 take the same state. Note that, in FIG. 61, the resistance values of resistors R11, R12, R21, and R22 are set so as to satisfy $V_{th1}$ > Vmiddle > $V_{th2}$.

Then, in the example, the two counters 52 output COUTH and COUTL. As illustrated in FIG. 62 that is an operation example of the sixth circuit configuration example, a counter value of the COUTH is incremented when both of the two magnetoresistive elements 10 are in the antiparallel state. In addition, a counter value of the COUTL is incremented when both of the two magnetoresistive elements 10 are in the parallel state. Accordingly, the stay time difference S explained above can be calculated using (COUTH - COUTL)/(COUTH + COUTL).

Note that the calculation explained above may be performed by a circuit in the magnetic detection device 20 or may be performed by an external processor after being output to the outside of the magnetic detection device 20.

FIG. 63 is the seventh circuit configuration example of the digitizing means according to the present embodiment. In FIG. 63, the digitizing means include a plurality of count units illustrated in FIG. 60 or FIG. 61. Then, in this example, COUT1, COUT2,..., and COUTk are sequentially selected by internal selection signals 1 to k and a result of accumulation by an accumulator (ACC) 54 is output to the vertical signal line 27 based on a row selection signal.

Note that, in the present embodiment, a circuit configuration of the digitizing means is not limited to the examples illustrated in FIG. 56 to FIG. 63.

As explained above, in the present embodiment, the direction of the easy axis of at least one magnetoresistive element 10 included in the pixel unit 25 is made different from the direction of the easy axes of the other magnetoresistive elements 10 included in the pixel unit 25. Alternatively, in the present embodiment, the direction of the easy axis of the magnetoresistive element 10 configuring at least one pixel unit 25 included in the basic array pattern 31 is made different from the direction of the easy axes of the magnetoresistive elements 10 configuring the other pixel units 25 included in the basic array pattern 31. In this way, according to the present embodiment, the magnitudes of the input magnetic field B in two or more axial directions can be detected with high sensitivity at a matched position in a space. Furthermore, in the present embodiment, since the pixel unit 25 and the basic array pattern 31 are arranged in an array to configure the pixel array 24, it is possible to implement the magnetic detection device 20 having spatial/temporal resolution while having a simpler structure.

Furthermore, by including a plurality of the magnetic detection devices 20 in the present embodiment, it is possible to measure a magnetic field in a wider spatial range. For example, a magnetocardiogram can be measured by disposing the plurality of magnetic detection devices 20 on the chest of a human. Alternatively, a magnetoencephalogram can be measured by disposing the plurality of magnetic detection devices 20 on the head of a human.

<<2. Second embodiment>>

<2.1 First configuration example of decoding system>

Subsequently, a decoding system that decodes a thought (in the present specification, the term "thought" includes imagining, intention, recognition, judgment, and the like by a human or an animal) of a human or an animal (referred to as a user) using the magnetic detection device 20 according to the first embodiment of the present disclosure explained above is explained.

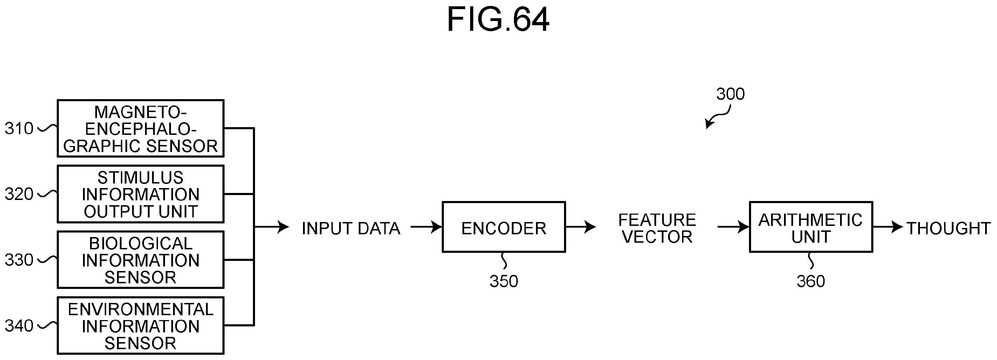

FIG. 64 is a configuration example of a decoding system according to a second embodiment of the present disclosure. As illustrated in FIG. 64, a decoding system 300 according to the present embodiment includes a magnetoencephalographic sensor 310 capable of measuring a magnetoencephalogram of a human or an animal using the magnetic detection device 20 according to the first embodiment. The decoding system 300 includes a stimulus information output unit 320 that outputs information concerning a stimulus given to a human or an animal, a biological information sensor 330 that acquires biological information concerning a human or an animal, and an environmental information sensor 340 that outputs information concerning an environment around a human or an animal. Furthermore, the decoding system 300 includes an encoder 350 that calculates a feature vector based on input data that has been input, and an arithmetic unit 360 that estimates information concerning thought of a human or an animal based on the feature vector.

The magnetoencephalographic sensor 310 can measure a magnetoencephalogram of a human or an animal using the magnetic detection device 20 according to the first embodiment. The magnetoencephalographic sensor 310 is worn on the head of a human or an animal, measures the magnetoencephalogram, and inputs a measurement result to the encoder 350 explained below as input data.

The stimulus information output unit 320 inputs information concerning a stimulus given to a human or an animal such as visual, auditory, and tactile stimuli to the encoder 350 explained below as input data. For example, the stimulus information output unit 320 may be a display device or an acoustic device viewed by a human or an animal. In this case, the stimulus information output unit 320 inputs, to the encoder 350, image data or acoustic data to be output. Alternatively, for example, concerning stimulus information concerning the sense of touch, the sense of taste, the sense of smell, the hearing, or the like, a human inputs subjective information concerning the sense of touch on an object that the human is touching, the sense of taste that the human is tasting, and the hearing that the human is smelling to the stimulus information output unit 320. The stimulus information output unit 320 inputs the input information to the encoder 350.

The biological information sensor 330 can be a myoelectric sensor, a heart rate sensor, a pulse sensor, a blood flow sensor, a blood pressure sensor, a respiration sensor, an electroencephalogram sensor, a perspiration sensor, a skin temperature sensor, a skin conductivity sensor, or the like worn on a part of the body of a human or an animal. The biological information sensor 330 may be a motion sensor that detects a motion and a posture of the body of a human or an animal. Specifically, the motion sensor unit includes an acceleration sensor, a gyro sensor, and a geomagnetic sensor. Furthermore, the biological information sensor 330 may be an imaging device that captures a facial expression, a line of sight, or a movement of eyeballs of a human or an animal. Then, the biological information sensor 330 inputs sensing data by the various sensors explained above to the encoder 350 explained below as input data.

The environmental information sensor 340 may include a position information sensor such as a GPS (Global Positioning System) receiver that acquires position information of a human or an animal. The environmental information sensor 340 may include various other sensors such as a sound collection microphone, an atmospheric pressure sensor, a temperature sensor, and a humidity sensor in order to acquire environmental information indicating a state of an environment around a human or an animal. Then, the environmental information sensor 340 inputs sensing data by the above-described various sensors to an encoder 350 explained below as input data.

Note that the input data from the magnetoencephalographic sensor 310, the stimulus information output unit 320, the biological information sensor 330, and the environmental information sensor 340 may be time-series data and a format and the like of the input data are not particularly limited.

The encoder 350 acquires the input data from the magnetoencephalographic sensor 310, the stimulus information output unit 320, the biological information sensor 330, and the environmental information sensor 340, calculates a feature value such as a feature vector from the input data, and inputs a calculation result to the arithmetic unit 360 explained below. For example, the encoder 350 may calculate a feature value from the input data referring to a model obtained in advance by machine learning or may calculate a feature value by performing statistical processing (mean, variance, normalization, and the like) on the input data. The encoder 350 may include a preprocessing unit that preprocesses the input data.

The arithmetic unit 360 converts (decodes) the feature vector into a thought of a human or an animal. For example, the arithmetic unit 360 may convert the feature vector or the like into an indicator indicating a thought of a human or an animal or the like referring to a model (a mathematical expression or the like) obtained in advance by machine learning. The model obtained in advance by the machine learning can be obtained, for example, by inputting the feature vector obtained from the input data and subjectivity of a human or a behavior of a human or an animal linked with the input data to a supervised learning device such as a support vector regression or a deep neural network as an input signal and a training signal and causing the supervised learning device to perform machine learning. Note that such machine learning can be performed by incorporating a learning device into the configuration in FIG. 64.

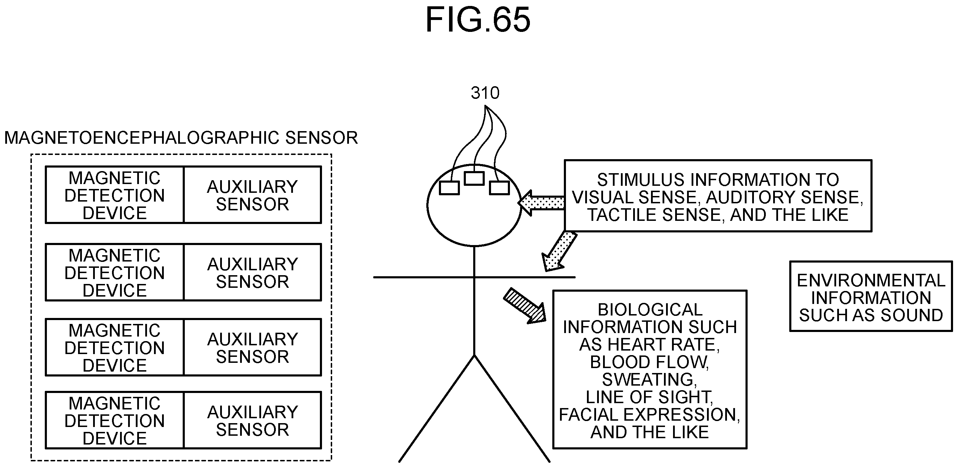

FIG. 65 is a use example of a first configuration of the decoding system according to the present embodiment. As illustrated in FIG. 65, a plurality of magnetoencephalographic sensors 310 are worn on the head of a human. The magnetoencephalographic sensor 310 includes a plurality of the magnetic detection devices 20 according to the first embodiment and may further include pluralities of the biological information sensors 330 and the environmental information sensors 340 as auxiliary sensors. Then, in the present embodiment, input data including at least one output of an output of the magnetoencephalographic sensor 310, an output from the stimulus information output unit 320, an output of the biological information sensor 330, and an output of the environmental information sensor is input to the encoder 350. The output from the stimulus information output unit 320 is information concerning a stimulus such as a visual, auditory, or tactile stimuli given to a human. The output of the biological information sensor 330 is biological information such as a heartbeat, a blood flow, sweating, a line of sight, and a facial expression of a human. The output from the environmental information sensor 340 includes information such as sound around a human. Then, the input data is converted into a feature vector by the encoder 350. Furthermore, the feature vector is converted into a thought of a human by the arithmetic unit 360. Note that, in the present embodiment, the input data includes at least the output of the magnetoencephalographic sensor 310.

Since it is possible to estimate a thought of a human or an animal based on a magnetoencephalogram of the human or the animal using the decoding system 300 explained above, it is possible to construct, for example, an automatic control device such as a robot that estimates a desire, which is one of thoughts of the human or the animal, and operates according to the estimated request. Such an automatic control device can operate in accordance with a request of a human even when the human has an impairment in skills (utterance, hand movement, and the like) for communicating the request to the outside. Therefore, the automatic control device can support the life of the human.

Since it is possible to estimate a thought of a human or an animal based on a magnetoencephalogram of the human or the animal using the decoding system 300 explained above, for example, it is possible to estimate an emotion, which is one of thoughts of the human, by giving such a stimulus (For example, an image or music). Then, based on the estimation, for example, a proposal of music or the like evoking joy can be suitably proposed according to an individual human.

<2.2 Second configuration example of decoding system>

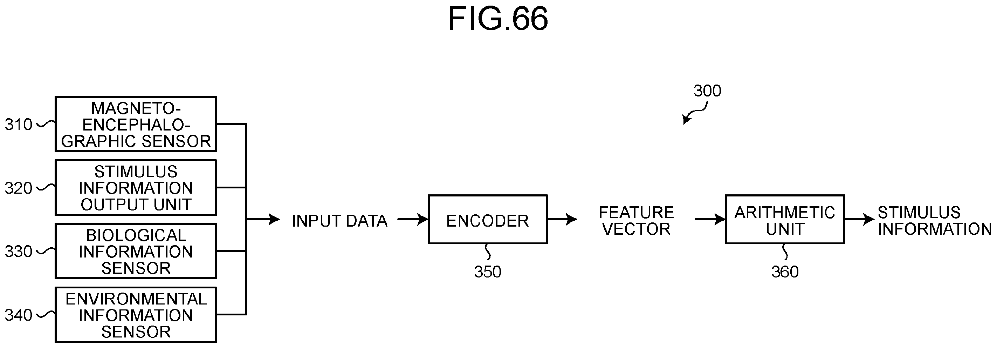

The decoding system may be a decoding system that generates stimulus information given to a human or an animal. FIG. 66 illustrates another configuration example of the decoding system according to the second embodiment of the present disclosure. In the configuration example, a decoding system that estimates a thought of a human or an animal and determines a stimulus given to the human according to the estimated thought using the magnetic detection device 20 according to the first embodiment of the present disclosure explained above is explained.

The decoding system 300 in the second configuration example basically has the same configuration as the first configuration example explained with reference to FIG. 64 but determines a stimulus (stimulus information) given to a human or an animal instead of estimating a thought of the human or the animal. The determined stimulus can be, for example, music, a video, an image, a scent, a feel, a dish, a product (daily necessities, clothing, or the like), or a schedule proposal given to a human or an animal.

The arithmetic unit 360 converts a feature vector into information concerning a stimulus given to a human or an animal (decoding). For example, the arithmetic unit 360 may convert the feature vector or the like into information concerning a stimulus of a human or an animal referring to a model (a mathematical expression or the like) obtained in advance by machine learning. The model obtained in advance by the machine learning can be obtained by, for example, inputting, to a supervised learning device such as a support vector regression or a deep neural network, as a training signal, input data obtained when a specific stimulus (music, a video, an image, a scent, a dish, or a commodity) is given and causing the supervised learning device to perform machine learning. Note that such machine learning can be performed by incorporating a learning device into the configuration in FIG. 66.

For example, although a stimulus felt comfortable varies depending on an individual human or animal, by using the present configuration example, it is possible to determine an appropriate stimulus that the individual human or animal feels comfortable. Therefore, by using the decoding system 300 explained above, for example, a proposal of comfortable music or the like can be suitably proposed according to an individual human. Although an appropriate stimulus that brings about a behavior modification is different depending on an individual human or animal, by using the present configuration example, it is possible to determine an appropriate stimulus for the individual human or animal and effectively urge a behavior modification.

Note that, in the present embodiment, the magnetoencephalographic sensor 310 can be mounted on a wearable device such as a HMD (Head Mounted Display) or a headphone worn on the head of a human.

<<3. Supplement>>

The preferred embodiment of the present disclosure is explained in detail above with reference to the accompanying drawings. However, the technical scope of the present disclosure is not limited to such an example. It is evident that those having the ordinary knowledge in the technical field of the present disclosure can arrive at various alterations or corrections within the category of the technical idea described in claims. It is understood that these alterations and corrections naturally belong to the technical scope of the present disclosure.

The effects described in the present specification are only explanatory or illustrative and are not limiting. That is, the technique according to the present disclosure can achieve other effects obvious for those skilled in the art from the description of the present specification together with or instead of the effects explained above.

Note that the present technique can also take the following configurations.

(1) A magnetic detection device comprising

a pixel array unit configured by pixel units including a magnetoresistive element and a detection unit being arranged in an array or configured by a basic array including a plurality of the pixel units being arranged in an array, wherein

the magnetoresistive element includes:

a fixed layer having a fixed magnetization direction;

a nonmagnetic layer disposed on the fixed layer; and

a storage layer disposed on the nonmagnetic layer, and

the detection unit detects an external magnetic field based on a change in a resistance value of the magnetoresistive element.

(2) The magnetic detection device according to (1), wherein

the storage layer includes an easy axis to which a magnetization direction is more easily directed than other directions and a difficult axis to which a magnetization direction of the storage layer is less easily directed than other directions, and

the detection unit

outputs, in linkage with coordinate information on the pixel array unit of the pixel unit corresponding to the detected magnetoresistive element, a detection result of the external magnetic field based on a change in a resistance value of the magnetoresistive element in a predetermined direction determined by a magnetization direction of the fixed layer of the magnetoresistive element of the pixel unit, and

in the pixel unit or the basic array, a direction of the easy axis of at least one of the magnetoresistive elements is different from a direction of easy axes of other magnetoresistive elements.

(3) The magnetic detection device according to (1) or (2), wherein

the magnetoresistive element

outputs, to the detection unit, at least one of information concerning a first stay time for maintaining a state in which a magnetization direction of the storage layer is parallel to a magnetization direction of the fixed layer and information concerning a second stay time for maintaining a state in which the magnetization direction of the storage layer is antiparallel to the magnetization direction of the fixed layer, and

the detection unit

detects the external magnetic field based on a difference between the first stay time and the second stay time specified based on at least one of the information concerning the first stay time and the information concerning the second stay time.

(4) The magnetic detection device according to any one of (1) to (3), further comprising:

a control unit that controls the magnetic detection device;

a vertical scanning unit that vertically scans the pixel array unit according to the control of the control unit; and

a horizontal scanning unit that horizontally scans the pixel array unit according to the control of the control unit.

(5) The magnetic detection device according to (3), wherein

the pixel unit includes a plurality of the magnetoresistive elements, and

the detection unit

detects the external magnetic field based on a difference between an integrated value of the first stay time and an integrated value of the second stay time in each of the magnetoresistive elements.

(6) The magnetic detection device according to any one of (1) to (4), wherein

the pixel unit

includes an array of magnetoresistive elements including a plurality of the magnetoresistive elements connected in parallel and/or in series, and
the detection unit
detects the external magnetic field based on a resistance value of the array of the magnetoresistive elements.

(7) The magnetic detection device according to any one of (1) to (4), wherein

the pixel unit includes a plurality of the magnetoresistive elements, and
the detection unit divides the plurality of the magnetoresistive elements included in the pixel unit corresponding thereto into a plurality of groups and detects the external magnetic field for each of the groups.

(8) The magnetic detection device according to (4), wherein
the horizontal scanning unit converts the external magnetic field detected for each of the pixel units into a digital value.

(9) The magnetic detection device according to any one of (1) to (7), wherein
the pixel unit further includes a conversion unit that converts the detected external magnetic field into a digital value.

(10) The magnetic detection device according to (2), wherein,
in at least one of the magnetoresistive elements, length in the easy axis direction is larger than length in the difficult axis direction.

(11) The magnetic detection device according to (10), wherein
a planar shape of at least one of the magnetoresistive elements is an elliptical shape.

(12) The magnetic detection device according to any one of (1) to (11), wherein
a planar shape of at least one of the magnetoresistive elements is a circular shape.

(13) The magnetic detection device according to (2), wherein
the pixel unit includes:

a first magnetoresistive element in which a direction of the easy axis is a first direction; and
a second magnetoresistive element in which a direction of the easy axis is a second direction different from the first direction by 90°.

(14) The magnetic detection device according to (13), wherein

the pixel unit
further includes a third magnetoresistive element in which a direction of the easy axis is a third direction different from each of the first direction and the second direction by 90°.

(15) The magnetic detection device according to (2), wherein
the basic array includes:

a first pixel unit including a first magnetoresistive element in which a direction of the easy axis is a first direction; and
a second pixel unit including a second magnetoresistive element in which a direction of the easy axis is a second direction different from the first direction by 90°.

(16) The magnetic detection device according to (15), wherein

the basic array
further includes a third pixel unit including a third magnetoresistive element in which a direction of the easy axis is a third direction different from each of the first direction and the second direction by 90°.

(17) The magnetic detection device according to (16), wherein
the basic array further includes:

a fourth pixel unit including the first magnetoresistive element and the third magnetoresistive element; and
a fifth pixel unit including the second magnetoresistive element and the third magnetoresistive element.

(18) The magnetic detection device according to (16) or (17), wherein

the basic array
further includes a sixth pixel unit including the first magnetoresistive element and the second magnetoresistive element.

(19) The magnetic detection device according to (18), wherein

the sixth pixel unit
further includes the third magnetoresistive element.

(20) A decoding system comprising: a magnetic detection device worn on a user; an encoder; and an arithmetic unit, wherein

the magnetic detection device
includes a pixel array unit configured by pixel units including a magnetoresistive element and a detection unit being arranged in an array or configured by a basic array including a plurality of the pixel units being arranged in an array,
the magnetoresistive element includes:

a fixed layer having a fixed magnetization direction;
a nonmagnetic layer disposed on the fixed layer; and
a storage layer disposed on the nonmagnetic layer,
the detection unit detects an external magnetic field based on a change in a resistance value of the magnetoresistive element,
the encoder
acquires the external magnetic field from the magnetic detection device as input data and
calculates a feature vector based on the input data, and
the arithmetic unit outputs, based on the feature vector, a thought of the user or a stimulus given to the user.

(21) The decoding system according to (20), wherein

the magnetic detection device
further includes a motion sensor that detects a motion and a posture of the user and
outputs an output of the motion sensor to the encoder together with the external magnetic field as the input data, and
the encoder
calculates the feature vector based on an output of the motion sensor.

(22) The decoding system according to (20) or (21), wherein

the magnetic detection device
further includes a biological information sensor that acquires biological information of the user and
outputs an output of the biological information sensor to the encoder together with the external magnetic field as the input data, and
the encoder
calculates the feature vector based on the output of the biological information sensor.

(23) The decoding system according to any one of (20) to (22), wherein
the input data includes information concerning at least one of an auditory stimulus, a visual stimulus, an olfactive stimulus, a taste stimulus, and a tactile stimulus given to the user.

Reference Signs List

10, 210C, 210E, 210L, 210NE, 210NW, 210V MAGNETORESISTIVE ELEMENT
11, 211, 216 MAGNETIZATION FIXED LAYER

12, 212, 217 NONMAGNETIC LAYER
13, 213, 218 STORAGE LAYER
20 MAGNETIC DETECTION DEVICE
21 CONTROL UNIT
22 VERTICAL SCANNING UNIT
23 HORIZONTAL SCANNING UNIT
24 PIXEL ARRAY
25, 25A, 25B, 25C, 25D PIXEL UNIT
26 ROW SELECTION SIGNAL LINE
27 VERTICAL SIGNAL LINE
28 DATA OUTPUT LINE
29 INFORMATION PROCESSING DEVICE
30 INPUT SIGNAL
31 BASIC ARRAY PATTERN
40 BASE SUBSTRATE
42, 243, 245 WIRE
50 ANALOG/DIGITAL CONVERTER
51 INTERFACE
52 COUNTER
53 COMPARATOR
54 ACCUMULATOR
214, 219 UPPER ELECTRODE
221, 222, 223, 224, 225, 226, 227, 228, 229, 230, 231 ARRAY
241, 242, 244 INSULATING LAYER
250C, 250E LAMINATED FILM
251, 256 FIRST LAYER
252, 257 SECOND LAYER
253, 258 THIRD LAYER
255C, 255E STRUCTURE
300 DECODING SYSTEM
310 MAGNETOENCEPHALOGRAPHIC SENSOR
320 STIMULUS INFORMATION OUTPUT UNIT
330 BIOLOGICAL INFORMATION SENSOR
340 ENVIRONMENTAL INFORMATION SENSOR
350 ENCODER
360 ARITHMETIC UNIT
A21, A23 TRENCH
A22, A24 OPENING
B1 BUFFER
D1 DIFFERENTIAL AMPLIFIER CIRCUIT
F1 FILTER MEANS
M21, M23 MASK
R1, R2, R4, R11, R12, R21, R22 RESISTOR
SH1 SAMPLING HOLD UNIT
SW1 ROW SELECTION SWITCH
SW2 MAGNETORESISTIVE SELECTOR SWITCH
SW3 SAMPLING HOLD PRE-READ RESET SWITCH

**Claims**

1. A magnetic detection device comprising

   a pixel array unit configured by pixel units including a magnetoresistive element and a detection unit being arranged in an array or configured by a basic array including a plurality of the pixel units being arranged in an array, wherein
   the magnetoresistive element includes:

a fixed layer having a fixed magnetization direction;
a nonmagnetic layer disposed on the fixed layer; and
a storage layer disposed on the nonmagnetic layer, and
the detection unit detects an external magnetic field based on a change in a resistance value of the magnetoresistive element.

2. The magnetic detection device according to claim 1, wherein

the storage layer includes an easy axis to which a magnetization direction is more easily directed than other directions and a difficult axis to which a magnetization direction of the storage layer is less easily directed than other directions, and
the detection unit
outputs, in linkage with coordinate information on the pixel array unit of the pixel unit corresponding to the detected magnetoresistive element, a detection result of the external magnetic field based on a change in a resistance value of the magnetoresistive element in a predetermined direction determined by a magnetization direction of the fixed layer of the magnetoresistive element of the pixel unit, and
in the pixel unit or the basic array, a direction of the easy axis of at least one of the magnetoresistive elements is different from a direction of easy axes of other magnetoresistive elements.

3. The magnetic detection device according to claim 1, wherein

the magnetoresistive element
outputs, to the detection unit, at least one of information concerning a first stay time for maintaining a state in which a magnetization direction of the storage layer is parallel to a magnetization direction of the fixed layer and information concerning a second stay time for maintaining a state in which the magnetization direction of the storage layer is antiparallel to the magnetization direction of the fixed layer, and
the detection unit
detects the external magnetic field based on a difference between the first stay time and the second stay time specified based on at least one of the information concerning the first stay time and the information concerning the second stay time.

4. The magnetic detection device according to claim 1, further comprising:

a control unit that controls the magnetic detection device;
a vertical scanning unit that vertically scans the pixel array unit according to the control of the control unit; and
a horizontal scanning unit that horizontally scans the pixel array unit according to the control of the control unit.

5. The magnetic detection device according to claim 3, wherein

the pixel unit includes a plurality of the magnetoresistive elements, and
the detection unit
detects the external magnetic field based on a difference between an integrated value of the first stay time and an integrated value of the second stay time in each of the magnetoresistive elements.

6. The magnetic detection device according to claim 1, wherein

the pixel unit
includes an array of magnetoresistive elements including a plurality of the magnetoresistive elements connected in parallel and/or in series, and
the detection unit
detects the external magnetic field based on a resistance value of the array of the magnetoresistive elements.

7. The magnetic detection device according to claim 1, wherein

the pixel unit includes a plurality of the magnetoresistive elements, and
the detection unit divides the plurality of the magnetoresistive elements included in the pixel unit corresponding thereto into a plurality of groups and detects the external magnetic field for each of the groups.

8. The magnetic detection device according to claim 4, wherein
the horizontal scanning unit converts the external magnetic field detected for each of the pixel units into a digital value.

9. The magnetic detection device according to claim 1, wherein
the pixel unit further includes a conversion unit that converts the detected external magnetic field into a digital value.

10. The magnetic detection device according to claim 2, wherein,
in at least one of the magnetoresistive elements, length in the easy axis direction is larger than length in the difficult axis direction.

11. The magnetic detection device according to claim 10, wherein
a planar shape of at least one of the magnetoresistive elements is an elliptical shape.

12. The magnetic detection device according to claim 1, wherein
a planar shape of at least one of the magnetoresistive elements is a circular shape.

13. The magnetic detection device according to claim 2, wherein
the pixel unit includes:

a first magnetoresistive element in which a direction of the easy axis is a first direction; and
a second magnetoresistive element in which a direction of the easy axis is a second direction different from the first direction by 90°.

14. The magnetic detection device according to claim 13, wherein

the pixel unit
further includes a third magnetoresistive element in which a direction of the easy axis is a third direction different from each of the first direction and the second direction by 90°.

15. The magnetic detection device according to claim 2, wherein
the basic array includes:

a first pixel unit including a first magnetoresistive element in which a direction of the easy axis is a first direction; and
a second pixel unit including a second magnetoresistive element in which a direction of the easy axis is a second direction different from the first direction by 90°.

16. The magnetic detection device according to claim 15, wherein

the basic array
further includes a third pixel unit including a third magnetoresistive element in which a direction of the easy axis is a third direction different from each of the first direction and the second direction by 90°.

17. The magnetic detection device according to claim 16, wherein
the basic array further includes:

a fourth pixel unit including the first magnetoresistive element and the third magnetoresistive element; and
a fifth pixel unit including the second magnetoresistive element and the third magnetoresistive element.

18. The magnetic detection device according to claim 16, wherein

the basic array
further includes a sixth pixel unit including the first magnetoresistive element and the second magnetoresistive element.

19. The magnetic detection device according to claim 18, wherein

the sixth pixel unit

further includes the third magnetoresistive element.

20. A decoding system comprising: a magnetic detection device worn on a user; an encoder; and an arithmetic unit, wherein

the magnetic detection device
includes a pixel array unit configured by pixel units including a magnetoresistive element and a detection unit being arranged in an array or configured by a basic array including a plurality of the pixel units being arranged in an array,
the magnetoresistive element includes:

a fixed layer having a fixed magnetization direction;
a nonmagnetic layer disposed on the fixed layer; and
a storage layer disposed on the nonmagnetic layer,
the detection unit detects an external magnetic field based on a change in a resistance value of the magnetoresistive element,
the encoder
acquires the external magnetic field from the magnetic detection device as input data and
calculates a feature vector based on the input data, and
the arithmetic unit outputs, based on the feature vector, a thought of the user or a stimulus given to the user.

21. The decoding system according to claim 20, wherein

the magnetic detection device
further includes a motion sensor that detects a motion and a posture of the user and
outputs an output of the motion sensor to the encoder together with the external magnetic field as the input data, and
the encoder
calculates the feature vector based on an output of the motion sensor.

22. The decoding system according to claim 20, wherein

the magnetic detection device
further includes a biological information sensor that acquires biological information of the user and
outputs an output of the biological information sensor to the encoder together with the external magnetic field as the input data, and
the encoder
calculates the feature vector based on the output of the biological information sensor.

23. The decoding system according to claim 20, wherein
the input data includes information concerning at least one of an auditory stimulus, a visual stimulus, an olfactive stimulus, a taste stimulus, and a tactile stimulus given to the user.

# FIG.1

INPUT
MAGNETIC
FIELD

10

13
12
11

# FIG.2

210E

213

Y
Z ⊙ → X

213
212
211

Z
Y ⊗ → X

# FIG.3

210C

218

Y

Z⊙→X

100

218
217
216

Z

Y⊗→X

# FIG.4

C: 90°

B: 60°

A: 0°

Y

Z⊙→X

# FIG.5

# FIG.6

# FIG.7

210V

Y

Z⊙ →X

# FIG.8

210NW

Y

Z⊙ →X

# FIG.9

210NE

# FIG.10

210C

# FIG.11

# FIG.12

# FIG.13

223

210C    210C    210C

# FIG.14

224

210V    210L    210V    210L

# FIG.15

225

210V 210L 210NW 210NE 210V 210L 210NW 210NE

# FIG.16

226

210V 210C 210L 210V 210C 210L

EP 4 617 697 A1

# FIG.17

# FIG.18

# FIG.19

210V    210V    210V      229

210C    210C    210C

...

...

...

# FIG.20

210V    210L    210V      230

210C    210C    210C

...

...

...

# FIG.21

X AXIS 221

Y AXIS 222

Z AXIS 223

231

CIRCUIT BOARD

# FIG.22

253
252  250E
251
40

# FIG.23

214
213
212  210E
211
40

# FIG.24

A21

241

40

255E

# FIG.25

A21

241

40

255E    256  257  258

250C

# FIG.26

A21

M21

255E    216  217  218

210C

241

40

# FIG.27

A21

219

255E    210C

241

40

# FIG.28

242

40

255E          255C

# FIG.29

A22          A22

242

40

255E          255C

# FIG.30

# FIG.31

# FIG.32

A23  243

241

100

40

# FIG.33

258
257  250C
256

241

255E  243  40

FIG.34

M23

218
217  210C
216

241

40

255E

243

FIG.35

219  210C

244

241

40

255E

243

## FIG.36

## FIG.37

# FIG.38

EP 4 617 697 A1

# FIG.39

# FIG.40

24

25D
25C
221

25A
223

31

223 ) 222
25B

# FIG.41

24

25D
25C
229

25A
228

31

228 ) 229
25B

# FIG.42

24

226
227
230
231
25

# FIG.43

```
            ┌──────────────┐
            │    START     │
            └──────────────┘
                   │
                   ▼
   ┌─────────────────────────────┐
   │       TURN ON POWER         │──S101
   └─────────────────────────────┘
                   │
                   ▼
   ┌─────────────────────────────┐
   │ MAGNETIC FIELD DETECTION    │
   │ BY MTJ → RESISTANCE VALUE   │──S102
   │ CHANGE                      │
   └─────────────────────────────┘
                   │
                   ▼
   ┌─────────────────────────────┐
   │ GENERATE READ SIGNAL BASED  │
   │ ON CHANGE IN RESISTANCE     │──S103
   │ VALUE OF MTJ                │
   └─────────────────────────────┘
                   │
                   ▼
   ┌─────────────────────────────┐
   │ RECEIVE SYNCHRONIZATION     │──S104
   │ SIGNAL                      │
   └─────────────────────────────┘
                   │
                   ▼
   ┌─────────────────────────────┐
   │   INITIALIZE READ ROW #R    │──S105
   └─────────────────────────────┘
                   │
                   ▼
   ┌─────────────────────────────┐
   │       OUTPUT Header         │──S106
   └─────────────────────────────┘
                   │
                   ▼
   ┌─────────────────────────────┐
   │        SELECT ROW           │──S107
   └─────────────────────────────┘
                   │
                   ▼
   ┌─────────────────────────────┐
   │ PIXELS INCLUDED IN SELECTED │──S108
   │ ROW OUTPUT READ RESULT      │
   └─────────────────────────────┘
                   │
                   ▼
   ┌─────────────────────────────┐
   │ HORIZONTAL SCANNING UNIT    │
   │ SEQUENTIALLY SCANS VSL      │──S109
   │ BASED ON COLUMN NUMBER #C   │
   │ AND OUTPUTS DATA            │
   └─────────────────────────────┘
                   │
                   ▼
   ┌─────────────────────────────┐
   │          R←R+1              │──S110
   └─────────────────────────────┘
                   │
                   ▼
            S111
        ◇ R>M? ◇───── NO
           │ YES
           ▼
   ┌─────────────────────────────┐
   │       OUTPUT Footer         │──S112
   └─────────────────────────────┘
                   │
                   ▼
            ┌──────────────┐
            │     END      │
            └──────────────┘
```

# FIG.44

# FIG.45

```
                    ┌──────────────┐
                    │    START     │
                    └──────┬───────┘
                           ▼
        ┌─────────────────────────────────────┐
        │   RECEIVE SYNCHRONIZATION SIGNAL     │── S201
        └─────────────────┬───────────────────┘
                          ▼
        ┌─────────────────────────────────────┐
        │  MAGNETIC DETECTION BY MTJ →         │── S202
        │  RESISTANCE VALUE CHANGE             │
        └─────────────────┬───────────────────┘
                          ▼
        ┌─────────────────────────────────────┐
        │  GENERATE READ SIGNAL BASED ON       │── S203
        │  CHANGE IN RESISTANCE VALUE OF MTJ   │
        └─────────────────┬───────────────────┘
                          ▼
        ┌─────────────────────────────────────┐
        │         HOLD READ SIGNAL            │── S204
        └─────────────────┬───────────────────┘
                          ▼
        ┌─────────────────────────────────────┐
        │       INITIALIZE READ ROW #R         │── S205
        └─────────────────┬───────────────────┘
                          ▼
        ┌─────────────────────────────────────┐
        │          OUTPUT Header               │── S206
        └─────────────────┬───────────────────┘
                          ▼
        ┌─────────────────────────────────────┐
        │           SELECT ROW                 │── S207
        └─────────────────┬───────────────────┘
                          ▼
        ┌─────────────────────────────────────┐
        │  PIXELS INCLUDED IN SELECTED ROW     │── S208
        │       OUTPUT READ RESULT             │
        └─────────────────┬───────────────────┘
                          ▼
        ┌─────────────────────────────────────┐
        │   HORIZONTAL SCANNING UNIT           │── S209
        │  SEQUENTIALLY SCANS VSL BASED ON     │
        │  COLUMN NUMBER #C AND OUTPUTS DATA   │
        └─────────────────┬───────────────────┘
                          ▼
        ┌─────────────────────────────────────┐
        │            R←R+1                     │── S210
        └─────────────────┬───────────────────┘
                          ▼
                    ╱───────────╲   S211
                   ╱   R>M?      ╲─────── NO
                   ╲             ╱
                    ╲───────────╱
                          │ YES
                          ▼
        ┌─────────────────────────────────────┐
        │          OUTPUT Footer               │── S212
        └─────────────────┬───────────────────┘
                          ▼
                    ┌──────────────┐
                    │     END      │
                    └──────────────┘
```

# FIG.46

EP 4 617 697 A1

# FIG.47

| | | |
|---|---|---|
| Header | | |
| #R | #C | //Bx// |

M ROWS x N COLUMNS

⋮

| #R | #C | //Bx// |
| Footer | | |
| Header | | |
| #R | #C | ·By· |

M ROWS x N COLUMNS

⋮

| #R | #C | ·By· |
| Footer | | |
| Header | | |
| #R | #C | \\Bz\\ |

M ROWS x N COLUMNS

⋮

| #R | #C | \\Bz\\ |
| Footer | | |

## FIG.48

Header

| #R | #C | Bx |
| --- | --- | --- |

M ROWS x N COLUMNS

⋮

| #R | #C | Bx |
| --- | --- | --- |
| #R | #C | By |

M ROWS x N COLUMNS

⋮

| #R | #C | By |
| --- | --- | --- |
| #R | #C | Bz |

M ROWS x N COLUMNS

⋮

| #R | #C | Bz |
| --- | --- | --- |

Footer

## FIG.49

Header

| #R | #C | Bx | By | Bz |
| --- | --- | --- | --- | --- |

M ROWS x N COLUMNS

⋮

| #R | #C | Bx | By | Bz |
| --- | --- | --- | --- | --- |

Footer

# FIG.50

# FIG.51

# FIG.52

# FIG.53

# FIG.54

# FIG.55

# FIG.56

...... VSL ~27

ADC IF

DL DATA OUTPUT

23 50 51 28

# FIG.57

...... VSL ~27 50

ADC ADC ADC ...... ADC

IF

DL DATA OUTPUT

23 51 28

# FIG.58

# FIG.59

# FIG.60

COUNTER CONTROL SIGNAL
(SUPPLIED FROM CONTROL UNIT
BY SEPARATE SYSTEM)

CLK     RST

COUNTER
EN

COUT

52

53

VDD

R1

R2     R3

N1

N2

10

GND

# FIG.61

EP 4 617 697 A1

# FIG.62

| MTJ1 | MTJ2 | Vmtj | Vo$_1$ | Vo$_2$ | COUTH | COUTL |
|---|---|---|---|---|---|---|
| LARGE | LARGE | Vlow | H | H | COUNT Up | NOT CHANGED |
| LARGE | SMALL | Vmiddle | L | H | NOT CHANGED | NOT CHANGED |
| SMALL | LARGE | Vmiddle | L | H | NOT CHANGED | NOT CHANGED |
| SMALL | SMALL | Vhigh | L | L | NOT CHANGED | COUNT Up |

# FIG.63

# FIG.64

EP 4 617 697 A1

# FIG.65

EP 4 617 697 A1

MAGNETOENCEPHALOGRAPHIC SENSOR

| MAGNETIC DETECTION DEVICE | AUXILIARY SENSOR |
|---|---|
| MAGNETIC DETECTION DEVICE | AUXILIARY SENSOR |
| MAGNETIC DETECTION DEVICE | AUXILIARY SENSOR |
| MAGNETIC DETECTION DEVICE | AUXILIARY SENSOR |

310

STIMULUS INFORMATION TO VISUAL SENSE, AUDITORY SENSE, TACTILE SENSE, AND THE LIKE

BIOLOGICAL INFORMATION SUCH AS HEART RATE, BLOOD FLOW, SWEATING, LINE OF SIGHT, FACIAL EXPRESSION, AND THE LIKE

ENVIRONMENTAL INFORMATION SUCH AS SOUND

# FIG.66

## INTERNATIONAL SEARCH REPORT

| International application No. |
| --- |
| **PCT/JP2023/039519** |

**A.    CLASSIFICATION OF SUBJECT MATTER**

*G01R 33/09*(2006.01)i; *A61B 5/242*(2021.01)i; *G01R 33/02*(2006.01)i; *G01R 33/10*(2006.01)i; *H10N 50/10*(2023.01)i; *H10N 59/00*(2023.01)i

FI:    G01R33/09; G01R33/02 R; G01R33/10; H10N50/10 Z; H10N59/00; A61B5/242

According to International Patent Classification (IPC) or to both national classification and IPC

**B.    FIELDS SEARCHED**

Minimum documentation searched (classification system followed by classification symbols)

G01R33/09; A61B5/242; G01R33/02; G01R33/10; H10N50/10; H10N59/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
| --- | --- | --- |
| X | WO 2012/032962 A1 (KONICA MINOLTA INC) 15 March 2012 (2012-03-15) paragraphs [0001]-[0045], fig. 1- 11 | 1, 6, 7, 9 |
| Y | | 2, 4, 8, 10-23 |
| Y | JP 2011-512517 A (MAGIC TECHNOLOGIES, INC.) 21 April 2011 (2011-04-21) fig. 5-7, fig. 11 | 2, 4, 8, 10-19 |
| Y | JP 2005-32167 A (SONY CORP) 03 February 2005 (2005-02-03) paragraphs [0001]-[0223] | 20-23 |
| A | JP 2013-108923 A (TDK CORP) 06 June 2013 (2013-06-06) entire text, all drawings | 1-23 |
| A | WO 2017/115839 A1 (KONICA MINOLTA INC) 06 July 2017 (2017-07-06) entire text, all drawings | 1-23 |

✓ Further documents are listed in the continuation of Box C.          ✓ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **05 January 2024** | **23 January 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**

International application No.

**PCT/JP2023/039519**

| C. | DOCUMENTS CONSIDERED TO BE RELEVANT | |
|---|---|---|
| Category* | Citation of document, with indication, where appropriate, of the relevant passages | Relevant to claim No. |
| A | JP 2019-171019 A (ASAHI KASEI DENSHI KK) 10 October 2019 (2019-10-10)<br>entire text, all drawings | 1-23 |
| A | WO 2017/164229 A1 (TOHOKU UNIVERSITY) 28 September 2017 (2017-09-28)<br>entire text, all drawings | 1-23 |

Form PCT/ISA/210 (second sheet) (July 2022)

## INTERNATIONAL SEARCH REPORT
### Information on patent family members

International application No.

**PCT/JP2023/039519**

| Patent document cited in search report | | | Publication date (day/month/year) | Patent family member(s) | | | Publication date (day/month/year) |
|---|---|---|---|---|---|---|---|
| WO | 2012/032962 | A1 | 15 March 2012 | US | 2013/0165766 | A1 | |
| | | | | paragraphs [0001]-[0105], fig. 1- 11 | | | |
| | | | | EP | 2614770 | A1 | |
| | | | | CN | 103188992 | A | |
| JP | 2011-512517 | A | 21 April 2011 | WO | 2009/091589 | A1 | |
| | | | | fig. 5-7, fig. 11 | | | |
| | | | | US | 2009/0186770 | A1 | |
| JP | 2005-32167 | A | 03 February 2005 | (Family: none) | | | |
| JP | 2013-108923 | A | 06 June 2013 | US | 2013/0134969 | A1 | |
| | | | | entire text, all drawings | | | |
| WO | 2017/115839 | A1 | 06 July 2017 | US | 2019/0018083 | A1 | |
| | | | | entire text, all drawings | | | |
| | | | | EP | 3399324 | A1 | |
| | | | | CN | 108431620 | A | |
| JP | 2019-171019 | A | 10 October 2019 | US | 2019/0298202 | A1 | |
| | | | | entire text, all drawings | | | |
| WO | 2017/164229 | A1 | 28 September 2017 | US | 2019/0219633 | A1 | |
| | | | | entire text, all drawings | | | |
| | | | | KR | 10-2018-0125472 | A | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020156870 A